# EUROPÄISCHE PATENTSCHRIFT

(11) **EP 3 922 434 B1**
(45) Veröffentlichungstag und Bekanntmachung des Hinweises auf die Patenterteilung: **08.05.2024**
(21) Anmeldenummer: 21174257.2
(22) Anmeldetag: 18.05.2021
(51) Int. Cl.: B29C 53/04, B29C 53/10, B29C 57/00, A61B 46/10

(54) **VERFAHREN UND VORRICHTUNG ZUR HERSTELLUNG EINES ÜBERZUGS**
METHOD AND DEVICE FOR PRODUCING A COATING
PROCÉDÉ ET DISPOSITIF DE FABRICATION D'UN REVÊTEMENT

(30) Priorität: 20.05.2020 DE 102020113772; 09.11.2020 DE 102020129458
(43) Veröffentlichungstag der Anmeldung: 15.12.2021
(73) Patentinhaber: Plur, Andreas, 63571 Gelnhausen (DE)
(72) Erfinder: Plur, Andreas, 63571 Gelnhausen (DE)
(74) Vertreter: Stoffregen, Hans-Herbert

(56) Entgegenhaltungen:
- WO-A1-2011/059413
- FR-A1- 2 565 200

## Beschreibung

Die Erfindung ein Verfahren zur Anbringung einer Applikation an eine Lage eines Überzugs zum Schutz eines chirurgischen Instruments, insbesondere eines chirurgischen Roboterarms, nach dem Oberbegriff des Anspruchs 1 sowie eine Vorrichtung zur Durchführung der Verfahren nach dem Oberbegriff des Anspruchs 6.

Ein Verfahren und eine Vorrichtung der eingangs genannten Art sind in der WO 2011/059413 A1 beschrieben. Die Vorrichtung ist zum Teleskopfalten eines Überzugs aus einem faltbaren Material ausgebildet. Die Vorrichtung umfasst zumindest zwei Gruppen von Armen, durch die der Faltvorgang ausgeführt wird. Ferner sind horizontale Arme und vertikale Arme vorgesehen, die zum Öffnen und Schließen rechtwinklig zueinander interagieren. Ferner ist ein Längspositionierer vorgesehen, der mit den horizontalen Armen verbunden ist und Vorwärtsbewegungen in Richtung der vertikalen Arme in der Ebene vollzieht, in der diese zwischen die vertikalen Arme eingeführt werden. An den Gruppen von Armen sind rechteckförmige geschlossene Platten angeordnet, die jeweils in den schlauchförmigen Überzug eingreifen. Der Überzug weist keine Applikationen auf.

Die FR 2 565 200 A1 betrifft ein Verfahren und eine Vorrichtung zum Querfalten einer durchgehenden Längshülle, um eine plissierte Hülle zu erhalten. Die Falten der Hülle sind konzentrisch ringförmig und in einer Richtung senkrecht zur Längsachse der Hülle übereinander angeordnet. Die Vorrichtung umfasst einen stationären Rahmen, der aus mindestens drei vertikalen Säulen und mindestens drei horizontalen oberen Stangen besteht, die mit mindestens einer Führungsrolle für die flachgelegte Hülle ausgestattet sind. Ferner ist ein stationärer Schlitten vorgesehen, der eine Rolle für die flachgelegte Hülle aufnimmt sowie ein axiales Rohr, das vertikal in Verbindung mit einem Druckluftsystem angeordnet ist, dessen oberes freies Ende Luft befördert, um eine pseudozylindrische Hülle zu bilden. Des Weiteren sind Mittel vorgesehen zum Zusammendrücken der Hülle in einem Abstand, der im Wesentlichen der doppelten gewünschten Länge der Falten entspricht.

Die EP 0 371 909 A1 betrifft ein Verfahren wird der Folienüberzug auf einen, insbesondere zylinderförmigen Dorn aufgezogen oder in ein Rohr bzw. einen entsprechenden Körper mit dem die Einstecköffnung aufweisenden Ende voran eingelegt, wobei der Außendurchmesser von Dorn oder Rohr dem Innendurchmesser des Folienüberzugs und deren Länge mindestens der halben Länge des Folienüberzugs entspricht.

Anschließend wird das Ende mit der der Einstecköffnung gegenüberliegenden Ausgangsöffnung unter Umstülpen nach Außen gewendet. Ferner wird die Ausgangsöffnung koaxial verschoben, bis diese sich in etwa im Bereich der Einstecköffnung befindet. Hierzu wird ein weiteres Rohr mit erweitertem Durchmesser über das erste Rohr oder den Dorn unter Umstülpen über dieses Rohr verwendet.

Sodann erfolgt ein Wenden der der Einstecköffnung gegenüberliegenden neu gebildeten freien Öffnung, ein Umstülpen nach Außen sowie koaxiales Verschieben der freien Öffnung, bis diese sich in etwa im Bereich der Einstecköffnung befindet.

Obige Verfahrensschritte werden gegebenenfalls mehrfach sinngemäß wiederholt.

Das bekannte Verfahren ist nur anwendbar, wenn der Folienüberzug in Form eines Folienschlauchs eine vorbestimmte Länge aufweist. Zudem muss der Dorn mindestens der halben Länge des Folienüberzugs entsprechen. Bei der Verwendung eines Rohrs müssen Rohre mit verschiedenen Durchmessern eingesetzt werden, was mit erhöhtem Aufwand verbunden ist.

Die WO 2009/077176 A1 betrifft eine Folienschlauchabdeckung für medizinische Geräte, mit einem Folienschlauch, der einen offenen und einen geschlossenen Endabschnitt aufweist und der eine sterile und eine nichtsterile Seite aufweist. Dabei ist vorgesehen, dass die Folienschlauchabdeckung auf einer Außenseite des geschlossenen Endabschnitts auf der nichtsterilen Seite eine Klebefolie aufweist, an der das medizinische Gerät befestigt wird. Die Anbringung von Applikationen auf einen eine teleskopartige Steckfaltung aufweisenden Folienschlauch ist der WO 2009/077176 A1 nicht offenbart.

Davon ausgehend liegt der vorliegenden Erfindung die Aufgabe zugrunde, ein Verfahren und eine Vorrichtung zur Herstellung eines Überzugs wie Folienüberzugs mit teleskopartiger Steckfaltung bereitzustellen, welches einfach anwendbar ist und insbesondere ermöglicht, Applikationen auf dem Überzug in beliebiger Faltung anzubringen und eine Rückfaltung zu ermöglichen.

Die Aufgabe wird erfindungsgemäß u.a. durch ein Verfahren mit den Merkmalen des Anspruches 1 gelöst.

Das erfindungsgemäße Verfahren betrifft das Anbringen zumindest einer Applikation an einen eine teleskopartige Faltung aufweisenden Überzug.

Das Verfahren umfasst folgende Verfahrensschritte:
a) Aufschieben einer Faltung oder mehrerer Faltungen einer ersten Lage des Überzugs auf eine erste Falthilfe;
b) Fixieren der Faltung oder der Faltungen der ersten Lage mit einer zweiten Falthilfe;
c) Entfalten des Überzugs bis zu der fixierten Faltung oder den fixierten Faltungen;
d) Anbringen der zumindest einen Applikation an oder in die entfaltete Lage des Überzugs;
e) Rückfalten der ersten und zweiten Lage durch Einfalten der ersten Lage gegen einen Rand der ersten Falthilfe zur Bildung einer weiteren Faltung;
f) Öffnen der Fixierung und Abziehen der Faltungen von der ersten Falthilfe;
g) Aufschieben der Faltungen auf die erste Falthilfe und Fixieren der Faltungen;
h) Wiederholen der Verfahrensschritte e) bis g) bis der Schlauch rückgefaltet ist.

Das Verfahren ermöglicht die nachträgliche Anbringung von Applikationen in Form von Instrumentenaufnahmen, Durchfiihrungen, Aufklebern u.a. an einen bereits gefalteten Überzug auf besonders einfache und schnelle Weise. Nach dem Stand der Technik muss der Überzug händisch entfaltet und händisch rückgefaltet werden, was einerseits mit erhöhtem Zeitaufwand und andererseits mit der Gefahr einer Beschädigung verbunden ist.

Zur weiteren Vereinfachung der zuvor beschriebenen Verfahren ist vorgesehen, dass während des Faltens Luft in den Überzug bzw. den Schlauch eingeblasen wird.

Vorteilhaft ist auch, dass der Schlauch während des Faltens von einer Endlosrolle abgerollt wird. Folglich können Endlosschläuche verwendet werden.

Vorzugsweise wird zumindest eine der Falthilfen zum Öffnen und Schließen mechanisch, pneumatisch, elektrisch, magnetisch und/oder hydraulisch angetrieben und kann in beliebiger Position gehalten werden.

Ferner ist gemäß einer bevorzugten Ausführung vorgesehen, dass die Faltungen händisch oder automatisch mittels zumindest eines Greifers oder Mitnehmers durchgeführt werden. Dabei kann der zumindest eine Greifer oder Mitnehmer eine Lage des Schlauchs greifen und diese in den von den Falthilfen aufgespannten Raum einführen. Das Greifen kann vorzugsweise durch Klemmen oder durch Unterdruck in Form eines Sauggreifers erfolgen.

Besonders bevorzugt wir der Verfahrensschritt der Fixierung aus der Gruppe Klemmen durch relatives Verfahren der Fixierhilfen, Klemmen durch eine separate externe Klemmvorrichtung oder Anziehen der Lage durch Unterdruck an zumindest eine der Fixierhilfen ausgewählt.

Des Weiteren betrifft die Erfindung eine Faltvorrichtung zur Herstellung eines Überzugs mit teleskopartiger Faltung nach dem Oberbegriff des Anspruchs 6. Die Vorrichtung umfasst zumindest eine erste Falthilfe, in die und/oder über die ein Ende eines Schlauchs zur Herstellung der Faltung wiederholt abschnittsweise einsteckbar oder überstülpbar ist.

Gemäß der Erfindung ist vorgesehen, dass die Faltvorrichtung ein Fixiermittel aufweist, mittels dem das Ende des Schlauchs oder zumindest eine Faltung an der zumindest einen Falthilfe fixierbar ist.

Das Fixiermittel ist eine zweite Falthilfe, wobei die erste und zweite Falthilfe zum Fixieren des Schlauchs relativ zueinander verfahrbar sind.

Gemäß der Erfindung ist vorgesehen, dass die Faltvorrichtung zum Falten eines Applikationen aufweisenden Überzugs ausgebildet ist, wobei die erste und/oder zweite Falthilfe Gabel- oder U-förmig ausgebildet ist oder wobei die erste und/oder zweite Falthilfe an die Applikationen angepasste Aussparungen aufweist, um ausreichend Raum für die Applikationen des Überzugs im gefalteten Zustand zu bieten.

Dabei kann das Ende des Schlauchs oder zumindest eine Faltung vorzugsweise zwischen den Falthilfen oder an einer der Falthilfen fixiert werden, wobei der Schlauch durch eine Relativbewegung zwischen den Falthilfen und dem Schlauch gegen einen Anschlag zumindest einer der Falthilfen über die Falthilfen zur Herstellung der Faltung überstülpbar ist.

Bei einer alternativen Ausführung ist Ende des Schlauchs über das erste und/oder zweite Faltelement stülpbar, wobei die Falthilfen zum Öffnen des Endes zueinander spreizbar ausgebildet sind, wobei der Schlauch durch eine Relativbewegung zwischen den Falthilfen und dem Schlauch gegen zumindest einen Anschlag der ersten oder zweiten Falthilfe in einen von den Falthilfen aufgespannten Raum einsteckbar ist.

Die Vorrichtung ist konstruktiv einfach und kann von einer Bedienperson auf einfache Weise bedient werden, ohne dass die Gefahr einer Beschädigung des Schlauchs besteht.

Vorzugsweise sind die Falthilfen derart relativ zueinander verfahrbar, wobei das Ende des Schlauchs oder zumindest eine erste Faltung zwischen den Falthilfen fixierbar ist oder wobei das Ende des Schlauchs oder zumindest eine erste Faltung durch die Falthilfen spreizbar ist. Ein Verrutschen des Schlauchs oder der Faltungen auf den Falthilfen wird verhindert, so dass ein präzises und sicheres Falten ermöglicht wird

Eine weitere bevorzugte Ausführung zeichnet sich dadurch aus, dass zumindest eine der Falthilfen Saugmittel als das Fixiermittel aufweist. Vorzugsweise sind die Saugmittel als Mündungsöffnung in einer Oberfläche der zumindest einen Falthilfe ausgebildet. Die Mündungsöffnungen können über einen oder mehrere Kanäle mit einer Unterdruckquelle verbunden sein. Die Mündungsöffnungen können an beliebigen Positionen auf Unter- und Oberseite der Falthilfen verteilt über die Oberfläche angeordnet sein.

Die erste oder zweite Falthilfe ist mit einem Abtrieb gekoppelt, der vorzugsweise mechanisch, elektrisch, magnetisch, pneumatisch und/oder hydraulisch angetrieben und in beliebiger Position arretierbar ist.

Vorzugsweise umfasst die zumindest eine erste oder zweite U-förmige Falthilfe zwei Schenkel, mit jeweils einem äußeren Rand, wobei die äußeren Ränder parallel zueinander verlaufen und die Breite BK definieren, und wobei eine Länge der Schenkel und/oder der Abstand der Schenkel zueinander einstellbar ist.

Die Oberfläche der Falthilfen kann glatt, strukturiert und/oder beschichtet sein um die Haftungskräfte der Folien an den Falthilfen zu verbessern.

Zumindest eine der Falthilfen kann vorzugsweise auch einen teleskopierbaren Arbeitsabschnitt aufweisen, der zusammen mit einer Faltung ausziehbar ist. Der Arbeitsabschnitt kann als Arbeitsfläche zur Anbringung der Applikation dienen.

Die Falthilfe ist gemäß einer eigenständigen Erfindung als Applikationshilfe ausgebildet und weist ein oder mehrere Elemente auf, die jeweils für sich oder zusammengesetzt eine Arbeitsfläche bilden. Auf der Arbeitsfläche können Handlungen aus der Gruppe Drucken, Scannen, Detektieren, Beschneiden, Ausschneiden, Zuschneiden, Stanzen, Lochen, Perforieren und/oder Bekleben zumindest einer Folienschlauchbahn ausgeführt werden.

Beispielsweise kann die Falthilfe als Unterlage zum Aufbringen eines Drucks oder zum Beschneiden, Ausschneiden, Zuschneiden, Stanzen, Lochen, Perforieren und/oder Bekleben der zumindest einen Folienschlauchbahn ausgebildet sein.

Auch wird eine mechanische, physikalische und/oder chemische Behandlung einer Innen-oder Außenseite der Schlauchbahn ermöglicht. Die Applikationshandlungen können auch automatisiert ausgeführt werden.

Die zweite Falthilfe ist vorzugsweise als Fixierelement ausgebildet, mittels dem das Ende des Schlauchs oder zumindest eine der Faltungen gegen die erste Falthilfe fixierbar ist.

Die Schenkel erstrecken sich vorzugsweise von einem Querträger, wobei die Schenkel in dem Querträger entlang einer Längsachse dieses verschiebbar angeordnet sind. Dadurch kann die Falthilfe an verschiedenen Breiten von Folienschläuchen angepasst werden.

Ferner ist erfindungsgemäß vorgesehen, dass das erste und/oder zweite Faltelement entlang eines Stützelementes verfahrbar angeordnet sind, wobei zumindest eine der Falthilfen mit einem Antrieb gekoppelt ist, der vorzugsweise mechanisch, elektrisch, magnetisch, pneumatisch und/oder hydraulisch angetrieben und in beliebiger Position arretierbar ist.

Zur weiteren Vereinfachung des Faltvorgangs, insbesondere zur Ausweitung des Schlauchs ist vorgesehen, dass die Faltvorrichtung ein Mittel zur Erzeugung einer Luftströmung aufweist, wobei eine Luftströmungsöffnung derart ausgerichtet ist, um Luft in den zwischen den Faltelementen fixierten bzw. aufgespannten Enden des Schlauchs einzuführen.

Der Schlauch umfasst ein flexibles Material, welches aus der Gruppe Kunststofffolie, Papier, Vliesstoff, gewebter Stoff ausgewählt ist. Vorzugsweise ist der Schlauch ein Folienschlauch.

Ferner können die Falthilfen Markierungen aufweisen, über die verschiedene Falttiefen definiert werden. Die Markierungen können in Form eines Aufdrucks auf die Oberseite der Falthilfen in Form eines Maßstabs, in Form von haptischen Markierungen wie Einbuchtungen, Erhebungen sowie in Form von Anschlägen sowie als aktive optische Markierungen in Form von Leuchtbändern, Lasermarkierungen auf einer Ober- oder Unterseite der ersten und/oder zweiten Falthilfe ausgebildet sein.

Ergänzend können Signalgeber in Form von Fotozellen, Endschaltern oder dergleichen vorgesehen sein, die bei Erreichen einer vorgegebenen Falttiefe ein Signal in Form eines akustischen, optischen oder haptischen Signals erzeugen.

Um zu vermeiden, dass einzelne Faltungen oder die Bahnen des Folienschlauchs beim Fixieren zwischen den Falthilfen beschädigt werden, ist vorgesehen, dass auf einer Unterseite oberen Falthilfe ein oder mehrere Distanzelemente angeordnet sind, so dass beim Zusammenfahren der Falthilfen eine Fixierung erfolgt, ohne dass die Faltung bzw. eine Falte mit Kraft beaufschlagt wird.

Die Distanzelemente können aus einem flexiblen Material wie Silikon ausgebildet sein, z. B. in Form von punkt- oder streifenförmigen Elementen, so dass eine Fixierkraft nur an definierten Stellen auf die untere Falthilfe übertragen wird. Die Distanzelemente wirken auch als Abstandshalter zwischen den Falthilfen, um die Folienbahnen des Folienschlauchs zu schonen.

Je nach Stärke der Folienbahn können die Distanzelement aus einem härteren Material wie Hartkunststoff ausgebildet sein. Auch besteht die Möglichkeit, dass die Distanzelemente eine Spitze aufweisen, um die Folienbahn des Folienschlauchs formschlüssig zu fixieren.

Als weitere alternative Ausführungsform wird vorgeschlagen, dass in den sich gegenüberliegenden Oberflächen der ersten und zweiten Falthilfe zueinander korrespondierende Distanzelemente in Form von Erhebungen und Vertiefungen angeordnet sind, wobei in der Oberfläche der unteren Falthilfe z. B. Erhebungen und in der Oberfläche der oberen Falthilfe z. B. Vertiefungen ausgebildet sein können, die zueinander korrespondieren.

Bei einer weiteren bevorzugten Ausführungsform ist vorgesehen, dass die Vorrichtung zumindest eine dritte Falthilfe umfasst. Dadurch kann eine Vollautomatisierung des Faltverfahrens erreicht werden. Während der zum Teil gefaltete Schlauch noch zwischen der ersten und zweiten Falthilfe fixiert ist, fährt die dritte Falthilfe in axialer Richtung in den gefalteten Schlauch hinein. Sobald die dritte Falthilfe parallel zu der ersten und zweiten Falthilfe in den Schlauch eingefahren ist, kann die die Fixierung gelöst werden und die erste Falthilfe kann in axialer Richtung aus der Faltung herausgezogen werden. Anschließend kann die Faltung zwischen der dritten und zweiten Falthilfe fixiert werden.

Nachdem eine weitere Faltung um die dritte Falthilfe ausgeführt ist, kann die erste Falthilfe wieder in axialer Richtung in die Öffnung des Schlauchs eingefahren werden, während die dritte Falthilfe aus der Faltung in axialer Richtung herausgezogen wird. Der Vorgang kann sukzessive wiederholt werden, ohne dass der Schlauch bzw. die Schlauchfaltung händisch von den Falthilfen abgezogen werden muss.

Weitere Einzelheiten, Vorteile und Merkmale der Erfindung ergeben sich nicht nur aus den Ansprüchen, den diesen zu entnehmenden Merkmalen - für sich und/oder im Kombination -, sondern auch aus der nachfolgenden Beschreibung von den Zeichnungen zu entnehmenden bevorzugten Ausführungsbeispielen sowie deren Erläuterungen.

Es zeigen:
- Fig. 1:: eine schematische Darstellung einer ersten Ausführungsform einer Faltvorrichtung,
- Fig. 2a bis 2d:: Verfahrensschritte zur Herstellung einer Faltung mit der Vorrichtung gem. Fig. 1,
- Fig. 3a bis 3d:: Verfahrensschritte zum Ent- und Rückfalten eines Folienüberzugs zum Anbringen einer Applikation,
- Fig. 4:: eine schematische Darstellung einer zweiten Ausführungsform einer Faltvorrichtung zur Herstellung eines Folienüberzugs mit teleskopartiger Steckfaltung,
- Fig. 5a bis 5d:: Verfahrensschritte zur Herstellung einer Faltung mit der Vorrichtung gem. Fig. 4,
- Fig. 6:: eine schematische Darstellung einer dritten Ausführungsform einer Faltvorrichtung,
- Fig. 7a bis 7d:: Verfahrensschritte zur Herstellung einer Faltung mit der Vorrichtung gern. Fig. 6,
- Fig. 8:: eine schematische Darstellung einer vierten Ausführungsform einer Faltvorrichtung,
- Fig. 9a bis 9f:: verschiedene Ausfiihrungsformen von Falthilfen,
- Fig. 10a:: eine Seitenansicht der Falthilfen der Faltvorrichtung gemäß Fig. 1 mit Distanzelementen,
- Fig.10b:: eine Vorderansicht der Falthilfen gemäß Fig. 10a,
- Fig. 11a:: eine Seitenansicht der Faltenhilfen der Faltvorrichtung gemäß Fig. 4 mit Distanzelementen,
- Fig. 11b:: eine Vorderansicht der Falthilfen gemäß Fig. 10a,
- Fig. 12:: eine Seitenansicht einer Falthilfe mit verschiedenen Ausführungsformen von Distanzelementen,
- Fig. 13:: eine Seitenansicht einer Falthilfe mit Schaumstoffbezug als Distanzelement,
- Fig. 14:: eine Draufsicht einer Falthilfe der Faltvorrichtung gemäß Fig. 1 mit einer Vielzahl von Distanzelementen,
- Fig. 15:: eine Seitenansicht von Falthilfen mit Distanzelementen sowie Aussparungen für Applikationen,
- Fig. 16:: eine Draufsicht auf eine fünften Ausführungsform einer Faltvorrichtung,
- Fig. 17:: eine Seitenansicht der Faltvorrichtung gemäß Fig. 16,
- Fig. 18a - 18c:: Verfahrensschritte zur Herstellung einer Faltung mit der Vorrichtung gemäß Fig. 16 und 17,
- Fig. 19:: eine Vorderansicht der Faltvorrichtung gemäß Fig. 4 mit Saugelementen zum Erfassen des Folienschlauchs und
- Fig. 20a - 20c:: eine sechste Ausführungsform einer Faltvorrichtung.

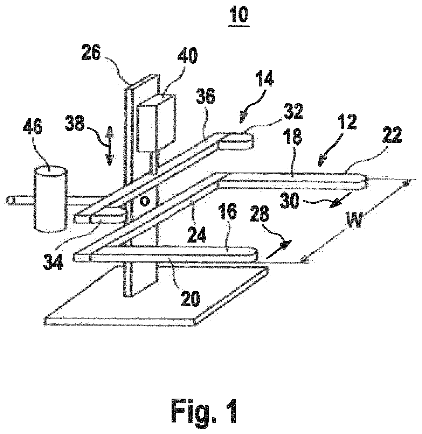

Fig. 1 zeigt rein schematisch eine eigenerfinderische erste Ausführungsform einer Faltvorrichtung 10, umfassend eine erste Falthilfe 12 und eine zweite Falthilfe 14. Die erste Falthilfe 12 ist gabelförmig ausgebildet, mit zwei Schenkeln 16, 18, die parallel zueinander verlaufen und jeweils einen Außenrand 20, 22 aufweisen, die parallel oder im Wesentlichen parallel verlaufen und eine Breite W aufspannen, die einer Breite des Folienschlauchs entspricht. Die Schenkel 16, 18 gehen von einem Querträger 24 aus, der an einer vertikalen Halterung 26 befestigt ist. Die Schenkel 16, 18 können vorzugsweise in Richtung der Pfeile 28, 30 entlang des Querträgers 24 auf eine gewünschte Breite W, die der Breite eines zu faltenden Folienschlauches entspricht, eingestellt werden.

Die zweite Falthilfe 14 ist als Fixierelement ausgebildet ist und weist eine gabelförmige Gestalt auf. Die zweite Falthilfe 14 umfasst Schenkel 32, 34, die von einer Schiene 36 ausgehen, die entlang der Halterung 26 in vertikaler Richtung entlang des Pfeils 38 über einen Antrieb 40 verfahrbar angeordnet ist. Die Schenkel 32, 34 sind im Vergleich zu den Schenkeln 16, 18 der Falthilfe 12 kürzer ausgebildet.

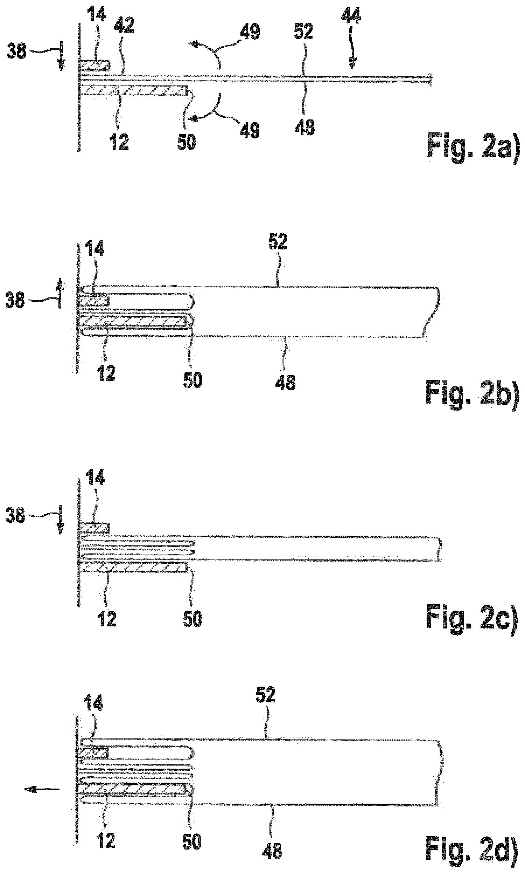

Ein Verfahren zur Herstellung einer teleskopartigen Steckfaltung unter Verwendung der Faltvorrichtung 10 gem. Fig. 1 ist anhand der Fig. 2a bis 2d erläutert. Ein Schlauchende 42 eines Folienschlauches 44 wird mittels der als Fixierelement ausgebildeten zweiten Falthilfe 14 gegen die erste Falthilfe 12 fixiert, wie dies in Fig. 2a dargestellt ist. Zur Aufweitung des Folienschlauchs 44 mittels Druckluft ist eine Druckluftpumpe 46 vorgesehen. Die eigeleitete Druckluft ist vorzugsweise sterilfiltriert, um Keimfreiheit zu gewährleisten.

Anschließend wird eine an der ersten Falthilfe 12 anliegende Lage 48 des Folienschlauches 44 in Richtung des Pfeils 40 um einen Anschlag 50 der ersten Falthilfe 12 umgelegt. Gleichzeitig wird eine obenliegende Lage 52 über das Schlauchende 42 gelegt, so dass eine erste Faltung ausgeführt wird, wie dies in Fig. 2b dargestellt ist.

Nach der Herstellung der ersten Faltung wird das Fixierelement 14 in Richtung des Pfeils 38 verfahren, um das Schlauchende 42 zu lösen.

Fig. 2c zeigt, wie das gefaltete Ende zwischen das Fixierelement 14 und die erste Falthilfe 12 eingespannt wird, wobei das Fixierelement 14 in Richtung des Pfeils 38 verfahren wird. Sodann wird die an der Falthilfe 12 anliegende Lage 48 um den Anschlag 50 umgelegt und eine weitere Faltung hergestellt, wie dies in Fig. 2d dargestellt ist. Die Verfahrensschritte 2a und 2b werden sukzessive so lange wiederholt, bis die gewünschte Anzahl von Faltungen hergestellt ist.

Neben der erfindungsgemäßen Herstellung von Folienüberzügen mit teleskopartiger Faltung kann die Vorrichtung gem. Fig. 1 für ein eigenerfinderisches Verfahren verwendet werden, das sich auf die Anbringung von Applikationen auf eine beliebige Lage bzw. Faltung eines ungefalteten oder gefalteten Folienüberzugs bezieht.

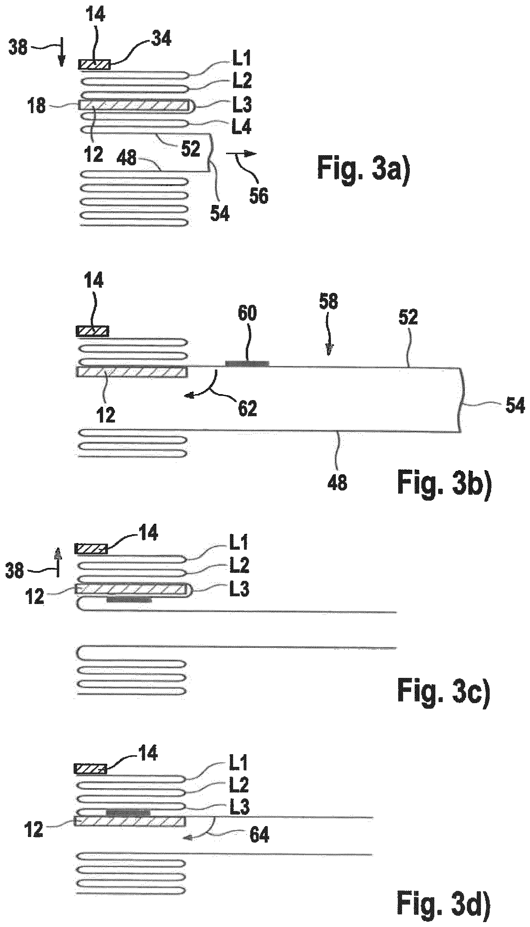

Fig. 3a zeigt einen gefalteten Folienüberzug in Form eines Schlauchpakets im Auslieferungszustand in Seitenansicht. Das Schlauchpaket wird z. B. mit der dritten Lage L3 der obenliegenden Lage 52 auf den Schenkel 18 der Falthilfe 12 durch eine Relativbewegung aufgeschoben, so dass die Lagen L1, L2 des Schlauchpaketes zwischen dem Schenkel 34 des Fixierelementes 14 und dem Schenkel 18 angeordnet sind. Anschließend wird das Fixierelement 14 in Richtung des Pfeils 38 verfahren, so dass die Lagen L1, L2 fixiert sind. Die untere Falthilfe 12 liegt unter der Lage L2, die gerade nicht mehr herausgezogen werden soll. Mit dem Fixierelement 14 werden die Lagen L1, L2, die über der Falthilfe 14 liegen, fixiert. Die Fixierung kann manuell, mechanisch, elektrisch, hydraulisch, magnetisch und/oder pneumatisch in beliebigen Positionen erfolgen.

In Fig. 3b ist der Verfahrensschritt dargestellt, wonach die nicht fixierten Lagen L3, L4 durch Ziehen eines Schlauchendes 54 in Richtung des Pfeils 56 herausgezogen werden.

Das Herausziehen kann über mehrere Faltungen hinweg erfolgen, wobei die geklemmten Lagen L1, L2 im Bündel zwischen den geschlossenen Gabeln 14, 18 verbleiben.

Der in Fig 3b dargestellte herausgezogene Schlauchabschnitt 58 kann nun bearbeitet werden, z. B. durch Aufbringen einer Applikation 60, z. B. einer Durchführung, eine Instrumentenhalterung, einen Aufkleber u.a.

Die gewünschten Lagen werden herausgezogen, wobei die Gegenlagen der fixierten Lagen im Bündel verbleiben und im Bündel in vertikaler Richtung nach unten durchhängen. Auf der ausgezogenen Folie werden die Applikationen angebracht.

Die Rückfaltung erfolgt, indem die erste Faltung, im vorliegenden Fall die Lage L3 unter den Schenkel 18 der Falthilfe 12 gemäß Pfeil 62 eingefaltet wird. Die Rückfaltung der unteren Lage erfolgt gleichzeitig, wie dies in Fig. 3c dargestellt ist. Die Faltung kann händisch oder mittels einer Einschubhilfe in Form eines Mitnehmers, einer Platte, einer Gabel oder dergleichen ausgeführt werden. Die Falthilfen sind Gabelförmig ausgebildet, um ausreichend Raum für die Applikation zu bieten. Auch können die Falthilfen Aussparungen aufweisen, die an die Applikationen angepasst sind.

Anschließend werden die Falthilfen 14, 12 in Richtung des Pfeils 38 geöffnet, so dass die Lagen L1, L2, L3 aus der Vorrichtung entnommen werden können. Die Lage L3 der rückgefalteten Lage 52 wird von der vormals geklemmten freien Seite geprüft und gegebenenfalls korrigiert.

Gemäß Fig. 3d wird das Bündel nun wieder auf die untere Gabel 12 geschoben. Die untere Gabel befindet sich nun unterhalb der im letzten Verfahrensschritt zurückgefalteten Folienlage L3 der oberen Bündellagen L1, L2, L3. Das Bündel aus den Lagen L1, L2, L3 wird mit der oberen Gabel 14 gegen die untere Gabel 12 fixiert. Die unteren Bündellagen hängen nach unten durch.

Das Bündel wird geklemmt und die Rückfaltung der nächsten Folienlage erfolgt nun wieder um die untere Gabel 12 herum in Richtung des Pfeils 64 entsprechend der Verfahrensschritte gem. Fig. 3a bis Fig. 3c. Der Vorgang wird so lange wiederholt, bis der Restschlauch eingefaltet ist. Die Klemmung wird geöffnet, das Bündel entnommen, korrigiert und zur nächsten Rückfaltung wieder so auf die untere Gabel 12 geschoben, dass die nun entstehende Rückfaltung wieder um die Gabel unten herum erfolgen kann.

Durch die Aussparungen in den Falthilfen 12, 14, insbesondere die gabelförmige Gestaltung der Falthilfen 12, 14 ist sichergestellt, dass das zerstörungsfreie Einfalten von Folienschläuchen mit angebrachten Applikationen ermöglicht wird; denn der Folienschlauch wird nur randseitig fixiert und die Applikationen können sich in dem von den Aussparungen gebildeten Raum frei ausbreiten ohne die Lage zu beschädigen.

Auf den Falthilfen 12, 14 können Markierungen angebracht sein, über die die Einfalttiefe definiert ist.

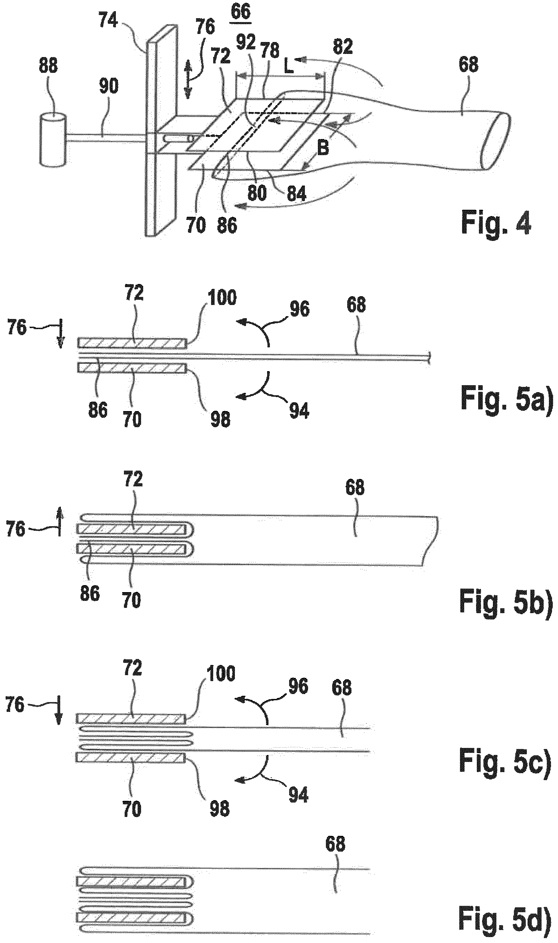

Fig. 4 zeig rein schematisch eine eigenerfinderische zweite Ausführungsform einer Faltvorrichtung 66 zum Falten eines Folienschlauchs 68 zur Herstellung eines Folienüberzugs mit teleskopartiger Steckfaltung. Die Vorrichtung 66 umfasst in dem dargestellten Ausführungsbeispiel eine erste Falthilfe 70 und eine zweite Falthilfe 72. Die Falthilfen 70, 72 sind jeweils in Form einer eine Ebene aufspannenden Platte ausgebildet. Die Platten weisen eine Breite B auf, die im Wesentlichen einer Breite des Folienschlauchs in einem flachen Zustand entspricht. Die Platten 70, 72 sind parallel zueinander an einer Halterung 74 angeordnet, wobei zumindest eine der Platten 70, 72 in vertikaler Richtung entlang der Halterung 74 verfahrbar angeordnet ist, so dass die Platten 70, 72 in eine geöffneten und eine geschlossene Stellung verfahrbar sind.

Die Falthilfen 70, 72 in Form von Platten weisen jeweils Randseiten 78, 80 bzw. 82, 84 auf, die in einem Abstand parallel zueinander verlaufen, der im Wesentlichen einer Breite BF des zusammengelegten Folienschlauchs 68 entspricht. Ferner weisen die Randseiten 78, 80, 82, 84 eine Länge auf, die im Wesentlichen einer maximalen Länge einer Faltung entspricht.

Im dargestellten Ausführungsbeispiel wird ein offenes Ende 86 des Folienschlauchs 68 zwischen die geöffneten Falthilfen 70, 72 eingelegt. Zum Öffnen des Endes 86 ist ein Druckluftreservoir 88 vorgesehen, mittels dem Druckluft über eine Leitung 90 in Richtung einer Öffnung 92 des Endes 86 eingebracht werden kann. Alternativ besteht auch die Möglichkeit, dass die Öffnung 92 des Folienschlauchs 68 manuell aufgeweitet wird.

Das Verfahren zur Herstellung des Folienüberzugs mit teleskopartiger Steckfaltung wird anhand der Fig. 5a bis 5d erläutert.

Gern. Fig. 5a wird das Ende 86 des Folienschlauchs 68 zwischen die Falthilfen 70, 72 eingelegt. Anschließend wird über das Druckluftreservoir 88 Druckluft in den Faltschlauch 68 eingeführt, um diesen aufzuweiten. Sodann wird die zweite Falthilfe 72 gegen die erste Falthilfe 70 verfahren, so dass das Ende 86 fixiert ist. Sodann werden die Lagen des Folienschlauchs 68 in Richtung der Pfeile 94, 96 koaxial um vordere Anschläge 98, 100 der Falthilfen 70, 72 zur Herstellung einer Faltung umgelegt bzw. übergestülpt.

Fig. 5b zeigt den Verfahrensschritt der ersten Faltung, wobei der Folienschlauch 68 einmalig über die Falthilfen 70, 72 übergestülpt ist. Das Schlauchende 86 ist zwischen den Falthilfen 70, 72 fixiert.

Nach Herstellung der ersten Faltung wird die zweite Falthilfe 72 in Richtung des Pfeils 76 geöffnet, so dass die Faltung aus der Faltvorrichtung 66 entnommen werden kann.

Fig. 5c zeigt einen weiteren Schritt, wobei der Abschnitt mit der ersten Faltung wieder zwischen die Falthilfen 70, 72 eingelegt und anschließend in die Falthilfe 72 in Richtung des Pfeils 76 gegen die Falthilfe 70 verspannt wird. Sodann wird der Folienschlauch wieder um die Anschläge 98, 100 umgestülpt.

Fig. 5d zeigt den Folienschlauch mit insgesamt drei Faltungen.

Die Verfahrensschritte 5a bis 5d können sukzessive wiederholt werden, bis die gewünschte Anzahl von Faltungen erreicht ist.

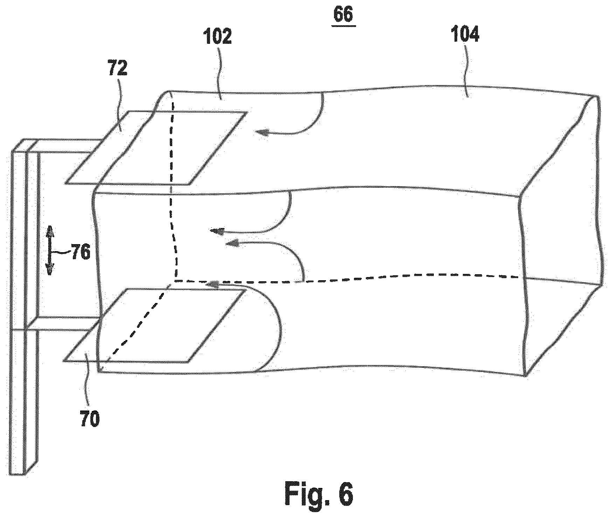

Fig. 6 zeigt die Vorrichtung 66 gem. Fig. 1 zur Durchführung einer alternativen Falttechnik. Im Gegensatz zu dem Verfahren gem. Fig. 5 wird ein Ende 102 eines Folienschlauchs 104 nicht zwischen die Falthilfen 70, 72 sondern nach Öffnung über die Falthilfen 70, 72 gestülpt. Anschließend werden die Falthilfen 70, 72 oder zumindest eine der Falthilfen 70, 72 in Richtung des Pfeils 76 verfahren, so dass der Folienschlauch 104 gespreizt wird.

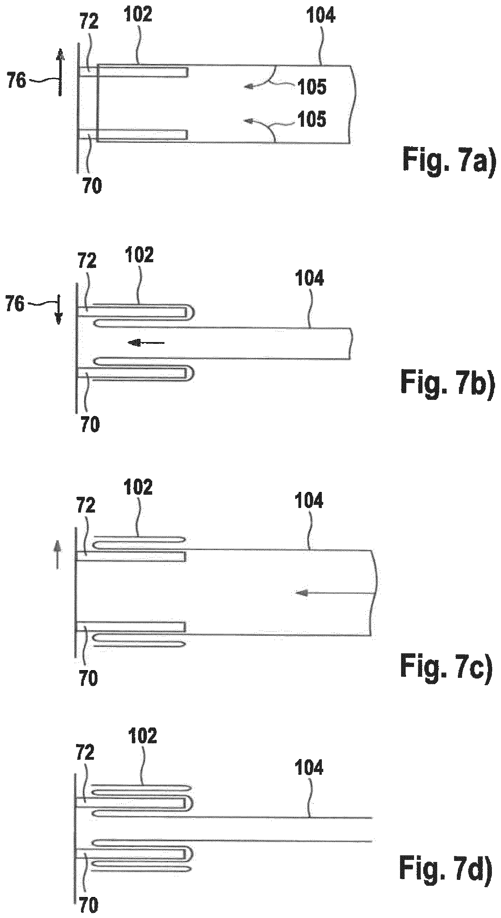

Das Verfahren zur Herstellung der Faltung wird gemäß Fig. 7a bis 7d erläutert.

Fig. 7a zeigt in Seitenansicht das Schlauchende 102, welches über die Falthilfen 70, 72 übergestülpt ist. In diesem Zustand sind die Falthilfen 70, 72 auseinandergefahren, so dass das Schlauchende 102 gespreizt ist.

In einem nächsten Verfahrensschritt, der in Fig. 7b dargestellt ist, wird die Lage des Folienschlauches 104 in Richtung der Pfeile 105 zwischen die Falthilfen 70, 72 nach innen eingefaltet. Nach dem Einfalten der Lage wird zumindest eine der Falthilfen 70, 72 in Richtung des Pfeils 76 verfahren, so dass die erste Faltung von den Falthilfen 70, 72 abgezogen werden kann.

In einem nächsten Verfahrensschritt, der in Fig. 7c dargestellt ist, wird das gefaltete Schlauchende wieder über die Falthilfen 70, 72 übergestülpt und zumindest einer Falthilfe in Richtung des Pfeils 76 nach außen gefahren, um das Schlauchende 102 zu fixieren.

In Fig. 7d ist dargestellt, wie das freie Schlauchende 102 nach innen zwischen die Falthilfen 70, 72 eingefaltet wird. Der Vorgang wird sukzessive so lange wiederholt, bis die gewünschte Anzahl an Faltungen erreicht ist.

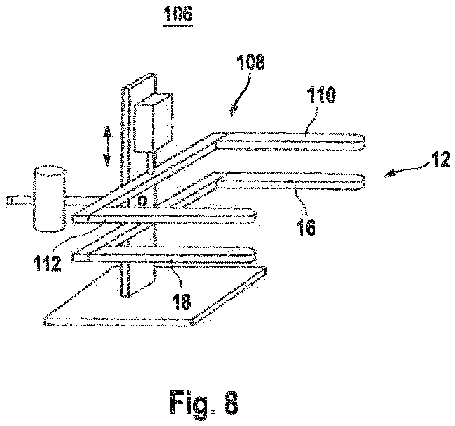

Fig. 8 zeigt rein schematisch eine vierte Ausführungsform einer Faltvorrichtung 106, die im Wesentlichen der Faltvorrichtung 10 gem. Fig. 1 entspricht, mit dem Unterschied, dass eine obere Falthilfe 108 in ihrer Gestaltung mit der unteren Falthilfe 12 korrespondiert. Die Falthilfe 108 ist ebenfalls gabelförmig ausgebildet ist, mit Schenkeln 110, 112, die im Wesentlichen eine Länge aufweisen, die den Schenkeln 16, 18 der Falthilfe 12 entspricht.

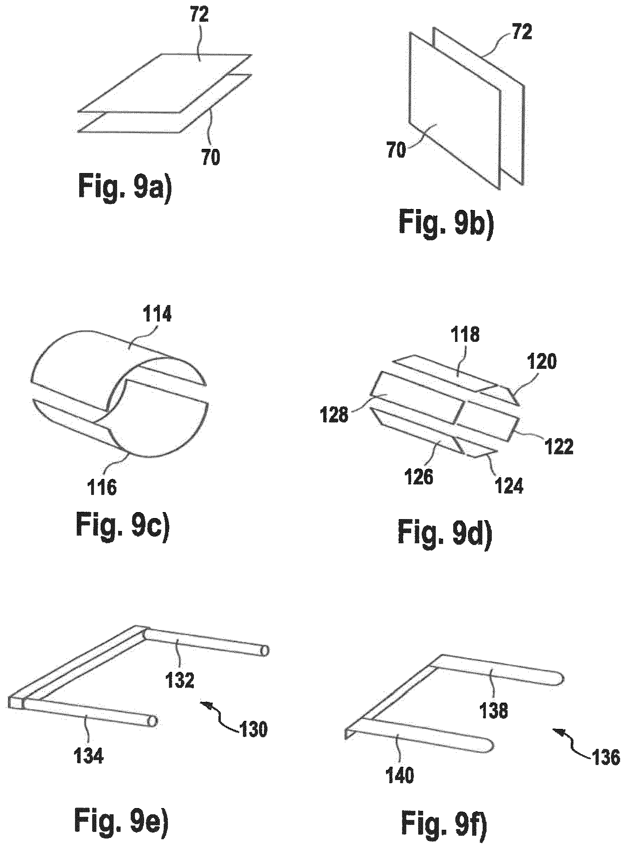

Fig. 9a bis 9f zeigt eine Vielzahl von Alternativen von Ausführungsformen von Falthilfen. Fig. 9a zeigt Falthilfen in Form von Platten 70, 72, wie diese in der Vorrichtung 66 nach Fig. 4 bzw. 6 verwendet werden.

Während die Platten 70, 72 gemäß Fig. 9a horizontal angeordnet sind, können diese auch wie in Fig. 9b dargestellt, in vertikaler Ausrichtung angeordnet sein.

Fig. 9c zeigt eine Ausführungsform von Falthilfen 114, 116 in Form von gebogenen Platten, die zusammen eine Zylinderform bilden und von denen zumindest eine in vertikaler Richtung verfahrbar ist, um ein Verspannen eines darauf aufgezogenen Schlauchendes zu ermöglichen.

Fig. 9d zeigt Falthilfen 118, 120, 122, 124, 126, 128, die im Querschnitt sechseckförmig angeordnet sind, von denen jedes einzelne in radialer Richtung verschiebbar angeordnet sein kann, um eine Spannfunktion zu ermöglichen.

Gemäß Fig. 9e zeigt eine Ausführungsform ebenfalls eine gabelförmige Falthilfe 130, wobei Schenkel 132, 134 in Form von Rohrelementen ausgebildet sind.

Fig. 9f zeigt eine Falthilfe 136, die ebenfalls gabelförmig ausgebildet ist, wobei die Schenkel 138, 140 in Form von Bügeln ausgebildet sind.

Zusammenfassend ist anzumerken, dass die erfindungsgemäßen Vorrichtungen 10, 66, 106 zumindest eine Falthilfe aufweist und zumindest ein Fixierelement. Die Falthilfen können je nach Anwendung eine ebene oder eine zylinderförmige Fläche aufspannen. Mögliche Ausführungen sind z. B. ebene oder gebogene Platten, U-förmige Bügel mit Schenkeln auch in Form eines Drahtgeflechtes. Die Falthilfen können Aussparungen in rechteckiger, halbrunder, runder oder mehreckiger Form aufweisen.

Ferner sind die Falthilfe und das Fixierelement relativ zueinander verfahrbar angeordnet, so dass eine Fixierung des Schlauchendes und/oder der Faltungen zwischen dem Fixierelement und der Falthilfe ermöglicht wird. Zudem wird ein Aufweiten eines über die Falthilfen gestülpten Schlauchendes ermöglicht.

Die Vorrichtung 10, 66, 106 kann so ausgebildet sein, dass das Schlauchende mechanisch oder automatisch auf bzw. zwischen die Falthilfen eingeschoben werden kann. Das Schlauchende kann auch durch zumindest einen Greifer mittels Unterdruck angesaugt und zwischen die Falthilfen eingezogen werden. Alternativ besteht auch die Möglichkeit, dass die Falthilfen in bzw. über das Schlauchende automatisch gestülpt bzw. eingefahren werden.

Der Antrieb 40 zur Betätigung des Fixierelementes kann mechanisch, elektrisch, pneumatisch, magnetisch, hydraulisch und/oder manuell ausgebildet sein. Die Falthilfen können aus Kunststoff, Metall und/oder Glas oder einem Verbundmaterial hergestellt sein. Zur besseren Haftung kann die Oberfläche der Falthilfe strukturiert und/oder beschichtet sein. In den Falthilfen können auch Kanäle eingebracht sein, die mit einer Unterdruckquelle verbunden sind. Die Kanäle können mit einer Mündungsöffnung in einer Oberfläche der Falthilfen enden und einen Unterdruck erzeugen, der die Folie fixiert. Der Unterdruck kann die Fixierung der Folien unterstützen oder auch die Klemmung durch zweite Falthilfen ersetzen.

Eine besonders bevorzugte Ausführungsform zeichnet sich dadurch aus, dass die Vorrichtung 10, 66, 106 mobil ausgebildet ist. Auch kann die Vorrichtung 10, 66, 106 insoweit automatisiert ausgebildet sein, dass die Falthilfen in das Schlauchende automatisch eingefahren werden.

Der zu faltende Schlauch kann aus einem flexiblen Material wie vorzugsweise Kunststofffolie, Papier, Tyvec, Vliesstoff, gewebten Stoff, z. B. Baumwolle, ausgebildet sein.

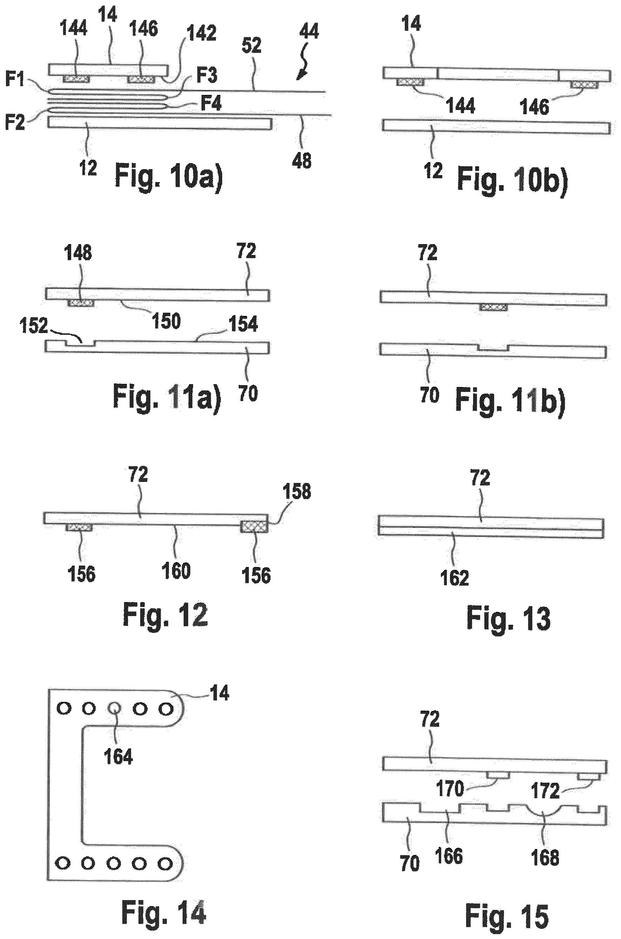

Fig. 10a und 10b zeigen eine Seiten- bzw. Vorderansicht von Falthilfen 12, 14 der Faltvorrichtung 10 gemäß Fig. 1.

Um zu vermeiden, dass einzelne Faltungen F1, F2, F3, F4 oder die Bahnen 52, 48 des Folienschlauchs 44 beim Fixieren zwischen den Falthilfen 12, 14 beschädigt werden, ist vorgesehen, dass auf einer Unterseite 142 der oberen, zweiten Falthilfe 14 ein oder mehrere Distanzelemente 144, 146 angeordnet sind, so dass beim Zusammenfahren der Falthilfen 12, 14 eine Fixierung erfolgt, ohne dass die Faltungen F1 - F4 mit Kraft beaufschlagt werden.

Die Distanzelemente 144, 146 können aus einem flexiblen Material wie Silikon ausgebildet sein, z.B. in Form von punkt- oder streifenförmigen Elementen, so dass eine Fixierung nur an definierten Stellen auf die untere, erste Falthilfe 12 übertragen wird. Die Distanzelemente 144, 146 wirken auch als eine Art Abstandshalter zwischen den Falthilfen 12, 14, um die Folienbahnen 44, 48 des Folienschlauchs 52 zu schonen.

Die Fig. 11a und 11b zeigen jeweils eine Seitenansicht sowie eine Vorderansicht der Falthilfen 70, 72 der Faltvorrichtung 66 gemäß Fig. 4. Bei dieser Ausführungsform ist vorgesehen, dass eine Falthilfe 148 auf einer Unterseite 150 der oberen Falthilfe 72 angeordnet ist, die in eine Ausnehmung 152 in der Oberfläche 154 der unteren Falthilfe 70 eingreift und somit eine Fixierung gewährleistet.

Fig. 12 zeigt eine Ausführungsform, wobei ein Distanzelement 156 in einer Ausnehmung 158 auf einer Unterseite 160 der oberen Falthilfe 72 fixiert ist. Das Distanzelement 156 kann alternativ auch direkt an der Unterseite 160 fixiert wie z. B. verklebt oder verschraubt sein. Eine magnetische Fixierung ist ebenfalls möglich, wodurch der Vorteil erreicht wird, dass die Distanzelemente 156 flexibel angeordnet und an verschiedene Schlauchformate und Applikationen angepasst werden können.

Fig. 13 zeigt eine weitere Ausführungsform eines Distanzelementes 162 in Form einer Schaumstoffbahn, insbesondere eines Würfel-Schaumstoffs, die auf der Unterseite 160 der oberen Falthilfe fixiert wie aufgeklebt ist. Je nach Anwendung können dann aus dem Würfel-Schaumstoff an gewünschter Position einzelne Würfel ausgebrochen bzw. herausgenommen werden, nämlich an den Positionen, an denen z.B. Applikationen auf dem zu faltenden Schlauch 44 aufgebracht sind.

Fig. 14 zeigt eine Unterseite der Falthilfe 14 der Faltvorrichtung gemäß Fig. 1 mit einer Vielzahl von Distanzelementen 164. Diese können beispielsweise als Stifte oder Stößel ausgebildet sein, die pneumatisch oder elektrisch verfahrbar sind, so dass eine Struktur der Distanzelemente 164 an verschiedene Applikationen angepasst werden kann.

Fig. 15 zeigt eine Seitenansicht der Falthilfen 70, 72 der Faltvorrichtung 66 gemäß Fig. 4, wobei in der unteren Falthilfe 70 Ausnehmungen 166, 168 ausgebildet sind, in denen z.B. Applikationen aufgenommen werden, die an dem Faltschlauch angebracht wurden. Die Aussparungen 166, 168 sind von Distanzelementen 170, 172 umgeben, um den gefalteten Schlauch im Bereich der Applikationen fixieren zu können. Dadurch wird erreicht, dass die Applikationen während des Faltvorgangs fixiert sind.

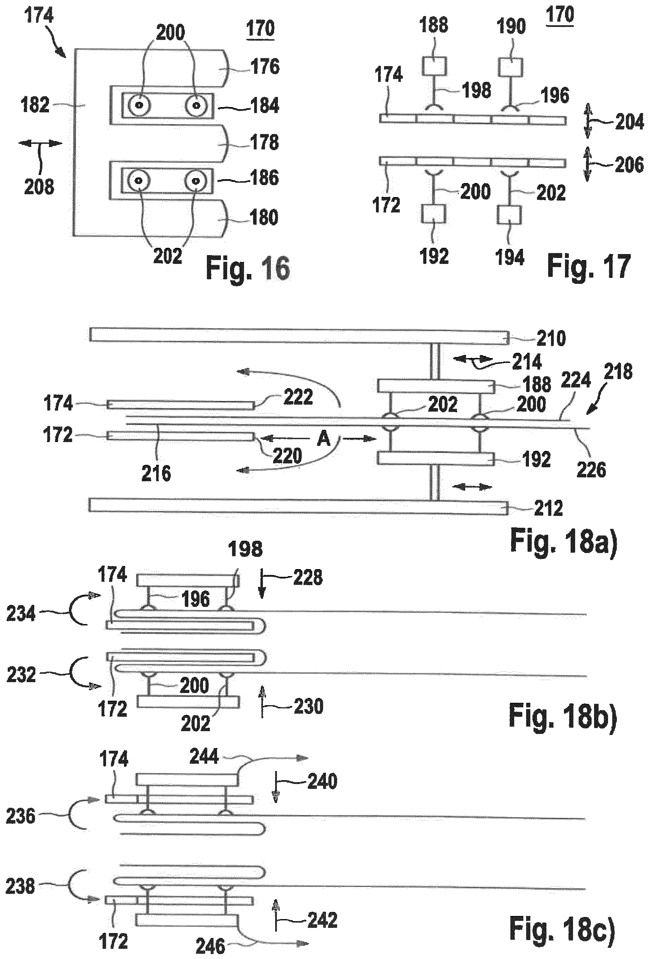

Die Fig. 16 und 17 zeigen eine fünfte Ausführung einer Faltvorrichtung 170 jeweils in Draufsicht (Fig. 16) und in Vorderansicht (Fig. 17). Die Faltvorrichtung 170 umfasst eine erste Falthilfe 172 und eine zweite Falthilfe 174, die Ebenen aufspannen, die parallel oder im Wesentlichen parallel zueinander verlaufen.

Fig. 16 zeigt die Falthilfen 172, 174 in einer Draufsicht, aus der ersichtlich ist, dass die Falthilfen jeweils E-förmig bzw. gabelförmig ausgebildet sind, mit Schenkeln 176, 178, 180, die randseitig über einen Steg 182 miteinander verbunden sind. Zwischen den Schenkeln 176,178 bzw. 178, 180 sind jeweils Aussparrungen 184, 186 ausgebildet, die sich bis zu dem Steg 182 verlaufen.

Gemäß einer eigenerfinderischen Ausführung weist die Faltvorrichtung 170 Greifelemente 188, 190, 192, 194 auf. Die Greifelemente 188 -194 weisen jeweils Saugelemente 196, 198, 200, 202 auf. Die Greifelemente 188, 190 sind in vertikaler Richtung oberhalb der zweiten Falthilfe 174 und die Greifelemente 192, 194 sind in vertikaler Richtung unterhalb der ersten Falthilfe 172 angeordnet.

Die Falthilfen 172, 174 sind sowohl jeweils einzeln in vertikaler Richtung entsprechend der Pfeile 204, 206 als auch jeweils einzeln in horizontaler Richtung entlang des Pfeils 208 beweglich angeordnet. Die Bewegungen können z. B. mittels einer Handhabungsvorrichtung wie Roboter oder eines Automaten ausgeführt werden, wobei die Falthilfen 172, 174 jeweils einzeln in horizontaler und vertikaler Richtung verfahrbar und ansteuerbar sind.

Auch die Greifelemente 188-194 mit den Saugelementen 196-202 sind sowohl in vertikaler Richtung als auch in horizontaler Richtung verfahrbar angeordnet. Die Handhabung der Greifelemente 188-194 kann über eine Handhabungseinrichtung wie Roboter oder automatisch entlang von Schienen 210, 212 ausgeführt sein.

Die Fig. 18a bis 18c zeigen einen Faltvorgang mittels der Faltvorrichtung 170. Im dargestellten Ausführungsbeispiel sind die Greifmittel 188-194 entlang von Schienen 210, 212 in Richtung des Pfeils 214 verfahrbar angeordnet, so dass diese über bzw. unter die Falthilfen 172, 174 verfahrbar sind. Dabei ist vorgesehen, dass die Greifmittel 188-194 derart verfahren werden, dass die Saugelemente 196-202 in die Aufnahmen 184, 186 zwischen den Stegen 176, 178, 180 platziert werden.

Fig. 18a zeigt einen Ausgangszustand, wobei ein Schlauchende 216 eines Schlauchs 218 zwischen den Falthilfen 172, 174 eingelegt und verspannt ist. Die Saugelemente 196, 202 sind in einem Abstand A vor einer Faltkante 220, 222 der Falthilfen 172, 174 angeordnet, wobei die Saugelemente 196-202 eine obere Lage 224 sowie eine untere Lage 226 des Folienschlauchs 218 greifen.

Sodann werden die Saugelemente 196, 202 entlang der Schienen 210, 212 in Richtung der Falthilfen 172, 174 verfahren, wie dies in Fig. 18b dargestellt ist. Dadurch wird eine erste Faltung hergestellt. Anschließend werden die Saugelemente 196-202 in Richtung der Pfeile 228, 230 verfahren, so dass mittels der Saugelemente 196-202 eine Fixierung der Faltung erzeugt wird. In einem weiteren Schritt werden die erste Falthilfe 172 und die zweite Falthilfe 174 in Richtung des Pfeils 232, 234 in horizontaler Richtung aus dem gefalteten Ende herausgezogen. In diesem Zustand ist die Faltung über die Saugelemente 196-202 fixiert.

In Fig. 18c ist ein weiterer Verfahrensschritt dargestellt, wobei die Faltelemente 172, 174 in Richtung der Pfeile 236, 238 von außen über die durch die Saugelemente 196, 202 fixierte Faltung geschoben werden. Die Saugelemente 196-202 sind in den Aussparungen 184-196 aufgenommen. Schließlich werden die Falthilfen 172, 174 jeweils in Richtung des Pfeils 240 bzw. 242 vertikaler verfahren, wobei das gefaltete Ende des Folienschlauchs dann wieder über die Falthilfen 172, 174 fixiert ist. Anschließend können die Saugelemente 196, 202 gelöst und jeweils in Richtung des Pfeils 244, 246 in die Ausgangsposition gem. Fig. 18a verfahren werden, so dass eine neue Faltung beginnen kann.

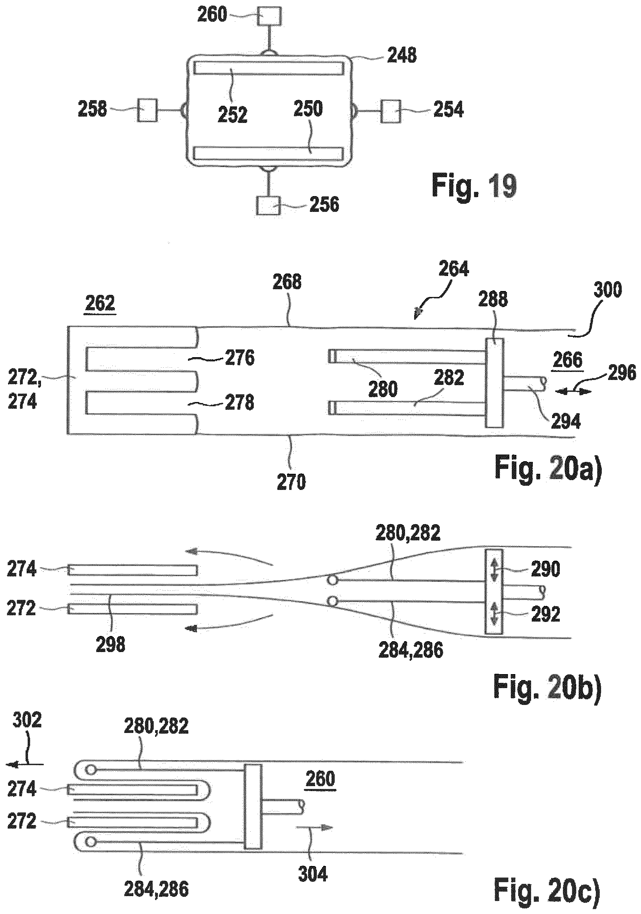

Fig. 19 zeigt eine Ausführungsform, wobei ein Schlauch 248 über eine erste Falthilfe 250 und eine zweite Falthilfe 252 übergestülpt ist. Bei dieser Ausführungsform können Saugmittel 254, 256, 258, 260 vorgesehen sein, die in einem Winkel von 90° entlang des Umfangs des Schlauchs 248 angeordnet sind.

Fig. 20a bis 20c zeigen eine weitere Ausführungsform einer Faltvorrichtung 262, wobei im Inneren eines Schlauchs 264 ein Faltwerkzeug 266 angeordnet ist, mittels dem die Lagen 268, 270 des Schlauchs 264 über Falthilfen 272, 274 gestülpt werden.

Fig. 20a zeigt eine Draufsicht der Faltvorrichtung 262. Die Falthilfen 272, 274 sind im Wesentlichen E-förmig ausgebildet, wie dies bereits mit Bezug zu Fig. 16 beschrieben wurde, mit jeweils frontseitig offenen Aussparungen 276, 278.

Das Faltwerkzeug 266 umfasst gem. Fig. 2a und 2b zwei Paare von Spreizelementen 280, 282 sowie 284, 286, die jeweils in einer Ebene angeordnet sind. Die Paare von Spreizelementen 280, 282; 284, 286 sind an einem Halteelement 288 befestigt und sind in Richtung des Pfeils 290, 292 zueinander verfahrbar angeordnet. Beim Auseinanderfahren der Spreizelemente 280, 282; 284, 286 wird der Folienschlauch gespreizt, wie dies in Fig. 20c dargestellt ist.

Das Halteelement 288 ist mit einem Schiebeelement 294 verbunden, mittels dem die Faltvorrichtung in Richtung des Pfeils 296 einerseits in die Aussparungen 276, 278 der Falthilfen 272, 274 zum Überstülpen des Schlauchs 268 und andererseits wieder zurück in eine Ausgangsstellung verfahrbar ist. Damit die Folienbahnen beim Spreizen und Stülpen durch die Spreizelemente nicht beschädigt werden, sind Enden der Spreizelemente abgerundet ausgebildet. Auch können Rollen (nicht dargestellt) an den Enden vorgesehen sein, wodurch ein Überstülpen der Folienbahnen über die Falthilfen weiter vereinfacht wird.

Die Vorrichtung funktioniert wie folgt. Zunächst wird ein Ende 298 des Schlauchs 264 zwischen den Falthilfen 272, 274 fixiert. Anschließend wird das Faltwerkzeug 266 über ein offenes Ende 300 des Schlauchs 268 in diesen eingeführt, wie dies in Fig. 20b dargestellt ist. Sodann werden die Spreizelemente 280, 282; 284, 286 in Richtung des Pfeils 290, 292 derart verfahren, dass der Schlauch 268 gespreizt wird.

In einem folgenden Schritt wird das Faltwerkzeug 260 in Richtung des Pfeils 296, d.h. in Richtung der Falthilfen 272, 274 verfahren, so dass die Folienbahnen 268, 270 über die Falthilfen 272, 274 gestülpt werden. Die Endstellung des Faltwerkzeugs 266 ist in Fig. 20c dargestellt.

Anschließend werden die Spreizelemente 280, 282; 284, 286 zueinander derart verfahren, so dass die Faltung durch die Spreizelemente 280, 282; 284, 286 fixiert ist. Die Falthilfen 272, 274 können dann in Richtung des Pfeils 302 aus der Faltung herausgefahren bzw. herausgezogen werden.

Anschließend werden dann die Falthilfen 272, 274 auseinandergefahren, über der Faltung platziert und zum Fixieren der Faltung wieder zusammengefahren. Dann kann die Fixierung durch die Spreizelement 280, 282; 284, 286 gelöst werden und das Faltwerkzeug 266 kann in Richtung des Pfeils 304 wieder aus der Faltung herausgezogen werden, so dass ein neuer Faltvorgang beginnen kann.

## Patentansprüche

1. Verfahren zum Anbringen zumindest einer Applikation (60) an einen eine teleskopartige Faltung aufweisenden Überzug (44), wie Folienüberzug, wobei das Verfahren folgende Verfahrensschritte umfasst:
a) Aufschieben einer Faltung oder mehrerer Faltungen (L1, L2, L3) einer ersten Lage (52) des Überzugs (44) auf eine erste Falthilfe (12);
b) Fixieren der Faltung oder der Faltungen der ersten Lage (L1, L2) vorzugsweise mit einer zweiten Falthilfe (14);
c) Entfalten des Überzugs (44) bis zu der fixierten Faltung oder den fixierten Faltungen (L1, L2) ;
d) Anbringen der zumindest einen Applikation (60) an oder in die entfaltete Lage (L3, L4) des Überzugs (44);
e) Rückfalten der entfalteten Lage oder Lagen (L3, L4) durch Einfalten der ersten Lage (52) gegen einen Rand der ersten Falthilfe (12) zur Bildung einer weiteren Faltung;
f) Öffnen der Fixierung und Abziehen der Faltungen (L1, L2, L3, L4) von der ersten Falthilfe (12);
g) Aufschieben der Faltungen (L1, L2, L3, L4) auf die erste Falthilfe (12) und Fixieren der Faltungen;
h) Wiederholen der Verfahrensschritte e) bis g) bis der Überzug (44) rückgefaltet ist.

2. Verfahren nach Anspruch 1,
**dadurch gekennzeichnet,**
**dass** während des Faltens Luft in den Überzug (44) bzw. den Schlauch (218) eingeblasen wird.

3. Verfahren nach Anspruch 1 oder 2,
**dadurch gekennzeichnet,**
**dass** zumindest eine der Falthilfen (12, 14) zum Öffnen und Schließen mechanisch, pneumatisch, elektrisch und/oder hydraulisch angetrieben und in beliebiger Position gehalten wird und/oder dass vorzugsweise die Faltungen (L1, L2, L3, L4) händisch oder automatisch mittels eines Greifers (188, 190, 192, 194) oder Mitnehmers durchgeführt werden, wobei der Greifer (188, 190, 192, 194) oder Mitnehmer vorzugsweise eine Lage des Schlauchs (218) durch Klemmen oder mittels Unterdruck erfasst und diese in den von den Falthilfen aufgespannten Raum einführt oder über die Falthilfen (12, 14) überstülpt.

4. Verfahren nach zumindest einem der vorhergehenden Ansprüche,
**dadurch gekennzeichnet,**
**dass** das Schlauchende (216) zwischen die erste und zweite Falthilfe (172, 174) eingelegt und durch Kraftbeaufschlagung der Falthilfen fixiert wird, dass eine erste und zweite Lage des Schlauchs (216) mittels eines erste und eines zweiten Greifelementes (188, 190, 192, 194) erfasst und über die erste und zweite Falthilfe durch Verfahren der ersten und zweiten Greifelement (188, 190, 192, 194) über die erste und zweite Falthilfe übergestülpt wird, dass die Faltung durch Kraftbeaufschlagung auf die Greifelemente (188, 190, 192, 194) fixiert wird, dass die Kraftbeaufschlagung der ersten und zweiten Falthilfe (172, 174) gelöst wird und dass die erste und zweite Falthilfe (172, 174) aus der Faltung herausgezogen werden, dass die erste und zweite Falthilfe (172, 174) derart verfahren werden, dass die Faltung zwischen der ersten und zweiten Falthilfe liegt und durch Kraftbeaufschlagung fixiert wird und dass die Greifelemente (188, 190, 192, 194) gelöst und zum erneuten Erfassen der erste und zweiten Lage des Schlauchs zur Durchführung einer erneuten Faltung verfahren werden.

5. Verfahren nach zumindest einem der vorhergehenden Ansprüche,
**dadurch gekennzeichnet,**
**dass** die Fixierung aus der Gruppe Klemmen durch relatives Verfahren der Fixierhilfen, relatives Verfahren der Greifer oder Mitnehmer, Klemmen durch separates Klemmelement oder Anziehen der Lage durch Unterdruck an zumindest eine der Fixierhilfen ausgewählt wird.

6. Faltvorrichtung (10; 66; 106) zur Herstellung eines Überzugs, wie Folienüberzugs, mit teleskopartiger Faltung, umfassend zumindest eine erste Falthilfe (12, 14; 70, 72; 108; 172, 174), in die und/oder über die ein Ende (42; 86; 102; 216) eines Schlauchs (44; 68; 104; 218) aus einem flexiblen Material zur Herstellung der Faltung wiederholt abschnittsweise einsteckbar oder überstülpbar ist, wobei die Faltvorrichtung (10; 66; 106) ein Fixiermittel (12, 14; 70, 72; 172, 174) aufweist, mittels dem das Ende (42; 86; 102; 216) des Schlauchs (44; 68; 104, 218) oder zumindest eine Faltung an der zumindest einen Falthilfe (12, 14; 70, 72; 108; 172, 174) fixierbar ist, wobei das Fixiermittel (14; 72; 108; 172, 174) eine zweite Falthilfe ist, wobei die erste und zweite Falthilfe (12, 14; 70, 72; 108; 172, 174) relativ zueinander verfahrbar sind,
**dadurch gekennzeichnet,**
**dass** die Faltvorrichtung (10; 66; 106) zum Falten eines Applikationen aufweisenden Überzugs (44) ausgebildet ist, wobei die erste und/oder zweite Falthilfe (12, 14; 70, 72; 108; 172, 174) Gabel- oder U-förmig ausgebildet sind oder wobei die erste und/oder zweite Falthilfe (12, 14; 70, 72; 108; 172, 174) an die Applikationen angepasste Aussparungen aufweisen, um ausreichend Raum für die Applikationen des Überzugs (44) im gefalteten Zustand zu bieten.

7. Faltvorrichtung nach Anspruch 6,
**dadurch gekennzeichnet,**
**dass** das Ende (42; 86; 216) des Schlauchs (44; 68, 218) oder die zumindest eine Faltung zwischen den Falthilfen (12, 14; 70, 72; 172, 174) oder an einer der Falthilfen (12, 14; 70, 72; 172, 174) fixierbar ist, wobei der Schlauch (44;68; 218) durch eine Relativbewegung zwischen den Falthilfen (12, 14; 70, 72; 172, 174) und dem Schlauch (44; 68; 216) gegen einen Anschlag (50; 220, 222) zumindest einer der Falthilfen (12, 14; 70, 72 ; 172, 174) über die Falthilfen (12, 14; 70, 72; 172, 174) zur Herstellung der Faltung überstülpbar ist und/oder dass vorzugsweise das Ende (102) des Schlauchs (104) über die erste und/oder zweite Falthilfe (70, 72) stülpbar ist, wobei die Falthilfen (70, 72) zum Öffnen des Endes (102) spreizbar ausgebildet sind, und wobei der Schlauch (104) durch eine Relativbewegung zwischen den Falthilfen (70, 72) und dem Schlauch (104) gegen zumindest einen Anschlag (98, 100) der ersten oder zweiten Falthilfe (70, 72) in einen von den Falthilfen aufgespannten Raum einsteckbar ist.

8. Faltvorrichtung nach Anspruch 6 oder 7"
**dadurch gekennzeichnet,**
**dass** die Falthilfen (12, 14; 70, 72; 172, 174) derart relativ zueinander verfahrbar sind, dass das Ende (42; 86; 216) des Schlauchs (44; 68; 218) oder zumindest eine erste Faltung zwischen den Falthilfen fixierbar ist oder dass das Ende (42; 86) des Schlauchs (44; 68) oder zumindest eine erste Faltung durch Spreizen der Falthilfen (70, 72) fixierbar ist und/oder dass vorzugsweise die zumindest eine Falthilfe (12, 14; 70, 72; 108; 172, 174) Unterdruckmittel als das Fixiermittel aufweist, die mit einer Unterdruckquelle verbunden sind, wobei die Unterdruckmittel als Mündungsöffnungen in einer Oberfläche der zumindest einen Falthilfe (12, 14; 70, 72; 108) ausgebildet sind, mittels denen der Schlauch oder eine Lage (48, 52) des Schlauchs fixierbar ist.

9. Faltvorrichtung nach zumindest einem der Ansprüche 6 bis 8,
**dadurch gekennzeichnet,**
**dass** die erste und/oder zweite Falthilfe (12, 14; 70, 72; 108; 172, 174) mit einem Antrieb (40, 88) gekoppelt ist, der vorzugsweise mechanisch, magnetisch, elektrisch, pneumatischen und/oder hydraulisch angetrieben und in beliebiger Position gehalten.

10. Faltvorrichtung nach zumindest einem der Ansprüche 6 bis 9,
**dadurch gekennzeichnet,**
**dass** die zumindest eine erste und/oder zweite U-förmig ausgebildete Falthilfe zwei Schenkel (16, 18; 32, 34; 110, 112) umfasst, mit jeweils einem äußeren Rand (20, 22), wobei die äußeren Ränder parallel zueinander verlaufen und die Breite W definieren, und wobei eine Länge der Schenkel (16, 18; 32, 34; 110, 112) und/oder der Abstand der Schenkel (16, 18; 32, 34; 110, 112) zueinander vorzugsweise teleskopierbar einstellbar ist.

11. Faltvorrichtung nach zumindest einem der Ansprüche 6 bis 10,
**dadurch gekennzeichnet,**
**dass** die Schenkel (16, 18; 32, 34; 110, 112) von einem Querträger (24; 36) ausgehen und dass die Schenkel (16, 18; 32, 34; 110, 112) in dem Querträger (24; 36) entlang einer Längsachse dieses verschiebbar angeordnet sind und/oder dass vorzugsweise die erste und/oder zweite Falthilfe (12, 14; 70, 72; 108) entlang eines Stützelementes (26; 74) verfahrbar angeordnet sind, wobei zumindest eine der Falthilfen (12, 14; 70, 72; 108) mit einem elektrischen, pneumatischen oder hydraulischen Antrieb (40) gekoppelt ist.

12. Faltvorrichtung nach zumindest einem der Ansprüche 6 bis 11,
**dadurch gekennzeichnet,**
**dass** die Faltvorrichtung (10) erste und zweite Greifelemente (188, 190; 192, 194) aufweist, die jeweils ausgebildet sind, eine erste und zweite Lage (224, 226) des Schlauchs (218) zu erfassen und dass die Greifelemente (188, 190; 192, 194) in Längsrichtung und/oder Querrichtung relativ zu der ersten und zweiten Falthilfe (172, 174) verfahrbar angeordnet sind, um die erste und zweite Lage (224, 226) des Schlauchs (218) über die Falthilfen (172, 174) zu stülpen.

13. Faltvorrichtung nach zumindest einem der Ansprüche 6 bis 12,
**dadurch gekennzeichnet,**
**dass** die erste und zweite Falthilfe (172, 174) jeweils zueinander korrespondierende Aussparungen (184, 186) aufweisen, die in Richtung eines Anschlags (220, 222) der ersten und zweiten Falthilfe (172, 174) offen, vorzugsweise schlitzförmig ausgebildet sind und/oder dass insbesondere die Aussparungen (184, 186) korrespondierend zu Greifern (196, 198; 200, 202) der Greifelemente (188, 190; 192, 194) ausgebildet sind und/oder dass insbesondere die Greifer (196, 198; 200, 202) gegeneinander verfahrbar ausgebildet sind, um die Lagen (224, 226) des Schlauchs (218) im Bereich der Aussparungen (184, 186) zu fixieren und/oder dass insbesondere die Falthilfen (172, 174) relativ zu den Greifelementen in Längs- und Querrichtung verfahrbar angeordnet sind und/oder dass insbesondere die Greifer als Sauggreifer ausgebildet sind.

14. Faltvorrichtung nach zumindest einem der Ansprüche 6 bis 13,
**dadurch gekennzeichnet,**
**dass** die Greifelemente (188, 190, 192, 194) entlang von Schienen (210, 212) relativ zu den Falthilfen (172, 174) insbesondere ober- bzw. unterhalb der Falthilfen (172, 174) verfahrbar angeordnet sind und/oder dass die Saugelemente (196, 198; 200, 202) gegeneinander verfahrbar angeordnet sind.

15. Faltvorrichtung nach zumindest einem der Ansprüche 6 bis 14,
**dadurch gekennzeichnet,**
**dass** die Faltvorrichtung ein Mittel (46; 88) zur Erzeugung einer Luftströmung aufweist, wobei eine Luftströmungsöffnung derart ausgerichtet ist, um die Luftströmung in das zwischen den Faltelementen (70, 72) fixierte bzw. aufgespannte Ende (42; 86; 102) des Folienschlauchs (44; 68; 104) einzuführen und/oder dass insbesondere der Schlauch aus einem flexiblen Material der Gruppe Kunststofffolie, Papier, Tyvec (eingetragene Marke), Vliesstoff, gewebter Stoff ausgewählt ist.

16. Faltvorrichtung nach zumindest einem der Ansprüche 6 bis 15,
**dadurch gekennzeichnet,**
**dass** die erste oder zweite Falthilfe (12, 14; 70, 72; 108) vorzugsweise eine Markierung aufweist, mittels der verschiedene Falttiefen definiert sind und/oder dass insbesondere die Markierung in Form eines Maßstabs auf einer Oberfläche der ersten oder zweiten Falthilfe aufgebracht ist und/oder dass die Markierung als haptische Markierung in Form einer seitlichen Kerbe oder oberflächenseitigen Nut ausgebildet ist und/oder dass die Markierung als optische Markierung in Form einer Leuchtdiode, eines Leuchtbandes und/oder einer Lasermarkierung ausgebildet ist.

17. Faltvorrichtung nach zumindest einem der Ansprüche 6 bis 16,
**dadurch gekennzeichnet,**
**dass** die erste und/oder zweite Falthilfe (12, 14; 70, 72, 108) auf einer der zweiten oder ersten Falthilfe (12, 14; 70, 72; 108) zugewandten Oberfläche zumindest ein Distanzelement aufweist, mittels dem das Ende des Schlauchs, die zumindest eine Lage des Schlauchs und/oder die zumindest eine Faltung des Schlauchs gegen die zweite oder erste Falthilfe fixierbar ist und/oder dass das insbesondere zumindest eine Distanzelement aus einem Material aus der Gruppe Silikon, Weichkunststoff, Hartkunststoff und/oder Metall ausgewählt ist und/oder dass das insbesondere zumindest eine Distanzelement vorzugsweise punkt-, streifenförmig oder wellenförmig ausgebildet ist und/oder dass das Distanzelement nadelförmig ausgebildet ist.

18. Faltvorrichtung nach zumindest einem der Ansprüche 6 bis 17,
**dadurch gekennzeichnet,**
**dass** die erste, zweite und/oder dritte Falthilfe mittels einer Handhabungseinrichtung wie Roboter geführt sind.

## Claims

1. A method for attaching at least one application (60) to a coating (44), such as a film coating, having telescopic folding,
wherein the method comprises the following method steps:
a) Pushing of one or more folds (L1, L2, L3) of a first layer (52) of the coating (44) onto a first folding aid (12);
b) Fixing of the fold(s) of the first layer (L1, L2) preferably using a second folding aid (14);
c) Unfolding of the coating (44) up to the fixed fold(s) (L1, L2);
d) Attachment of the at least one application (60) onto or into the unfolded layer (L3, L4) of the coating (44);
e) Folding back of the unfolded layer(s) (L3, L4) by folding in the first layer (52) against an edge of the first folding aid (12) to form a further fold;
f) Opening of the fixing and removal of the folds (L1, L2, L3, L4) from the first folding aid (12);
g) Pushing of the folds (L1, L2, L3, L4) onto the first folding aid (12) and fixing of the folds;
h) Repetition of method steps e) to g) until the coating (44) is folded back.

2. The method according to claim 1,
**characterized in**
**that** air is blown into the coating (44) or tube (218) during folding.

3. The method according to claim 1 or 2,
**characterized in**
**that** at least one of the folding aids (12, 14) is mechanically, pneumatically, electrically and/or hydraulically driven for opening and closing and is held in any position; and/or that the folds (L1, L2, L3, L4) are preferably made manually or automatically by means of a gripper (188, 190, 192, 194) or driver, wherein the gripper (188, 190, 192, 194) or driver preferably grips a layer of the tube (218) by clamping or by means of negative pressure and introduces said layer into the space created by the folding aid or fits it over the folding aids (12, 14).

4. The method according to at least one of the preceding claims,
**characterized in**
**that** the tube end (216) is inserted between the first and second folding aids (172, 174) and fixed by applying a force to the folding aids; that a first and second layer of the tube (216) are gripped by means of a first and second gripping element (188, 190, 192, 194) and fitted over the first and second folding aids by moving the first and second gripping elements (188, 190, 192, 194) over the first and second folding aids; that the fold is fixed by applying a force to the gripping elements (188, 190, 192, 194); that the force applied to the first and second folding aids (172, 174) is lifted and that the first and second folding aids (172, 174) are pulled out of the fold; that the first and second folding aids (172, 174) are moved such that the fold is located between the first and second folding aids and is fixed by applying a force; and that the gripping elements (188, 190, 192, 194) are released and moved to grip once again the first and second layers of the tube to perform further folding.

5. The method according to at least one of the preceding claims,
**characterized in**
**that** the fixing process is selected from the group clamping by relative movement of the fixing aids, relative movement of the grippers or drivers, clamping by a separate clamping element or tightening of the layer by negative pressure on at least one of the fixing aids.

6. A folding device (10; 66; 106) for producing a coating, such as a film coating, with telescopic folding, comprising at least one first folding aid (12, 14; 70, 72; 108; 172, 174), into which and/or over which an end (42; 86; 102; 216) of a tube (44; 68; 104; 218) made from a flexible material is repeatedly insertable or fittable section by section to make the fold, wherein the folding device (10; 66; 106) has a fixing means (12, 14; 70, 72; 172, 174) by means of which the end (42; 86; 102; 216) of the tube (44; 68; 104, 218) or at least one fold is fixable to the at least one folding aid (12, 14; 70, 72; 108; 172, 174), wherein the fixing means (14; 72; 108; 172, 174) is a second folding aid, and wherein the first and second folding aids (12, 14; 70, 72; 108; 172, 174) are movable relative to one another,
**characterized in**
**that** the folding device (10; 66; 106) is designed for folding a coating (44) having applications, wherein the first and/or second folding aids (12, 14; 70, 72; 108; 172, 174) are designed fork-shaped or U-shaped, or wherein the first and/or second folding aids (12, 14; 70, 72; 108; 172, 174) have cutouts adapted to the applications, to provide sufficient space for the applications on the coating (44) in the folded state.

7. The folding device according to claim 6,
**characterized in**
**that** the end (42; 86; 216) of the tube (44; 68, 218) or the at least one fold is fixable between the folding aids (12, 14; 70, 72; 172, 174) or on one of the folding aids (12, 14; 70, 72; 172, 174), wherein the tube (44; 68; 218) is, by a relative movement between the folding aids (12, 14; 70, 72; 172, 174) and the tube (44; 68; 216) against a stop (50; 220, 222) of at least one of the folding aids (12, 14; 70, 72; 172, 174), fittable over the folding aids (12, 14; 70, 72; 172, 174) to make the fold; and/or that preferably the end (102) of the tube (104) is fittable over the first and/or second folding aid (70, 72), wherein the folding aids (70, 72) are designed spreadable for opening of the end (102), and wherein the tube (104) is insertable into a space created by the folding aids by a relative movement between the folding aids (70, 72) and the tube (104) against at least one stop (98, 100) of the first or second folding aid (70, 72).

8. The folding device according to claim 6 or 7,
**characterized in**
**that** the folding aids (12, 14; 70, 72; 172, 174) are movable relative to one another such that the end (42; 86; 216) of the tube (44; 68; 218) or at least one first fold is fixable between the folding aids; or that the end (42; 86) of the tube (44; 68) or at least one first fold is fixable by spreading of the folding aids (70, 72); and/or that the at least one folding aid (12, 14; 70, 72; 108; 172, 174) preferably has negative pressure means as the fixing means, which are connected to a negative pressure source, wherein the negative pressure means are designed as discharge openings in a surface of the at least one folding aid (12, 14; 70, 72; 108), by means of which the tube or a layer (48, 52) of the tube is fixable.

9. The folding device according to at least one of claims 6 to 8,
**characterized in**
**that** the first and/or second folding aids (12, 14; 70, 72; 108; 172, 174) are coupled to a drive (40, 88) which is preferably mechanically, magnetically, electrically, pneumatically and/or hydraulically driven and held in any position.

10. The folding device according to at least one of claims 6 to 9,
**characterized in**
**that** the at least one first and/or second folding aid of U-shaped design comprises two arms (16, 18; 32, 34; 110, 112), with an outer edge (20, 22) respectively, wherein the outer edges extend parallel to one another and define the width W, and wherein a length of the arms (16, 18; 32, 34; 110, 112) and/or the distance of the arms (16, 18; 32, 34; 110, 112) to one another is preferably telescopically settable.

11. The folding device according to at least one of claims 6 to 10,
**characterized in**
**that** the arms (16, 18; 32, 34; 110, 112) extend from a transverse support (24; 36); that the arms (16, 18; 32, 34; 110, 112) in the transverse support (24; 36) are arranged slidable along a longitudinal axis thereof; and/or that the first and/or second folding aids (12, 14; 70, 72; 108) are preferably arranged movable along a supporting element (26; 74), wherein at least one of the folding aids (12, 14; 70, 72; 108) is coupled to an electric, pneumatic or hydraulic drive (40).

12. The folding device according to at least one of claims 6 to 11,
**characterized in**
**that** the folding device (10) has first and second gripping elements (188, 190; 192, 194) each designed to grip a first and a second layer (224, 226) of the tube (218); and that the gripping elements (188, 190; 192, 194) are arranged movable in the longitudinal direction and/or transverse direction relative to the first and second folding aids (172, 174) in order to fit the first and second layers (224, 226) of the tube (218) over the folding aids (172, 174).

13. The folding device according to at least one of claims 6 to 12,
**characterized in**
**that** the first and second folding aids (172, 174) respectively have cutouts (184, 186) corresponding to one another, which are designed open in the direction of a stop (220, 222) of the first and second folding aids (172, 174), preferably slot-like; and/or that in particular the cutouts (184, 186) are designed corresponding to grippers (196, 198; 200, 202) of the gripping elements (188, 190; 192, 194); and/or that the grippers (196, 198; 200, 202) are in particular designed movable against one another in order to fix the layers (224, 226) of the tube (218) in the area of the cutouts (184, 186); and/or that in particular the folding aids (172, 174) are arranged movable relative to the gripping elements in the longitudinal and transverse directions; and/or that the grippers are in particular designed as suction grippers.

14. The folding device according to at least one of claims 6 to 13,
**characterized in**
**that** the gripping elements (188, 190, 192, 194) are arranged movable along rails (210, 212) relative to the folding aids (172, 174) in particular above / below the folding aids (172, 174); and/or that the suction gripping elements (196, 198; 200, 202) are arranged movable against one another.

15. The folding device according to at least one of claims 6 to 14,
**characterized in**
**that** the folding device has a means (46; 88) for generating an air flow, wherein an air flow opening is aligned so as to introduce the air flow into the end (42; 86; 102) of the film tube (44; 68; 104) fixed / created between the folding elements (70, 72); and/or that in particular the tube is selected from a flexible material from the group plastic film, paper, Tyvec (registered trademark), non-woven material, woven material.

16. The folding device according to at least one of claims 6 to 15,
**characterized in**
**that** the first or second folding aid (12, 14; 70, 72; 108) preferably has a marking, by means of which different folding depths are defined; and/or that the marking is in particular applied in the form of a scale on a surface of the first or second folding aid; and/or that the marking is designed as a haptic marking in the form of a lateral notch or a groove on the surface side; and/or that the marking is designed as an optical marking in the form of an LED, a luminous strip and/or a laser marking.

17. The folding device according to at least one of claims 6 to 16,
**characterized in**
**that** the first and/or second folding aids (12, 14; 70, 72, 108) have on a surface facing said second or first folding aids (12, 14; 70, 72; 108) at least one spacer element, by means of which the end of the tube, the at least one layer of the tube and/or the at least one fold of the tube are fixable against the second or first folding aid; and/or that the in particular at least one spacer element is selected from a material from the group silicone, soft plastic, hard plastic and/or metal; and/or that the in particular at least one spacer element is designed preferably in dot, strip or wave form and/or that the spacer element is designed in needle form.

18. The folding device according to at least one of claims 6 to 17,
**characterized in**
**that** the first, second and/or third folding aids are guided by means of a handling device such as a robot.

## Revendications

1. Procédé pour fixer au moins une application (60) sur un revêtement (44), tel un film enveloppant, présentant un pliage télescopique,
sachant que le procédé comprend les étapes de procédé suivantes :
a) poussée d'un pliage ou de plusieurs pliages (L1, L2, L3) d'une première couche (52) du revêtement (44) sur un premier auxiliaire de pliage (12) ;
b) fixation du pliage ou des pliages de la première couche (L1, L2), de préférence avec un second auxiliaire de pliage (14) ;
c) dépliage du revêtement (44) jusqu'au pliage fixé ou aux pliages fixés (L1, L2) ;
d) fixation d'au moins une application (60) sur ou dans la couche dépliée (L3, L4) du revêtement (44) ;
e) repliage de la ou des couches dépliées (L3, L4) en pliant la première couche (52) contre un bord du premier auxiliaire de pliage (12) pour former un autre pliage ;
f) ouverture de la fixation et retrait des pliages (L1, L2, L3, L4) du premier auxiliaire de pliage (12) ;
g) poussée des pliages (L1, L2, L3, L4) sur le premier auxiliaire de pliage (12) et fixation des pliages ;
h) répétition des étapes e) à g) jusqu'à ce que le revêtement (44) soit replié.

2. Procédé selon la revendication 1,
**caractérisé en ce**
**que** pendant le pliage, de l'air est insufflé dans le revêtement (44) ou le tuyau (218).

3. Procédé selon la revendication 1 ou 2,
**caractérisé en ce**
**qu'**au moins un des auxiliaires de pliage (12, 14) est entraîné mécaniquement, pneumatiquement, électriquement et/ou hydrauliquement pour l'ouverture et la fermeture et maintenu dans une position quelconque et/ou que de préférence, les pliages (L1, L2, L3, L4) sont réalisés à la main ou automatiquement au moyen d'un préhenseur (188, 190, 192, 194) ou d'un entraîneur, sachant que le préhenseur (188, 190, 192, 194) ou l'entraîneur saisit de préférence une couche du tuyau (218) par pincement ou au moyen de dépression et l'introduit dans l'espace sous-tendu par les auxiliaires de pliage ou l'enfile sur les auxiliaires de pliage (12, 14).

4. Procédé selon au moins l'une des revendications précédentes,
**caractérisé en ce**
**que** l'extrémité du tuyau (216) est insérée entre le premier et le second auxiliaire de pliage (172, 174) et fixée aux auxiliaires de pliage par l'exercice d'une force, qu'une première et une seconde couche du tuyau (216) sont saisies au moyen d'un premier et d'un second élément de préhension (188, 190, 192, 194) et enfilées sur le premier et le second auxiliaire de pliage par déplacement du premier et du second élément de préhension (188, 190, 192, 194) sur le premier et le second auxiliaire de pliage, que le pliage est fixé par l'exercice d'une force sur les éléments de préhension (188, 190, 192, 194), que l'exercice de la force du premier et du second auxiliaire de pliage (172, 174) est déclenché et que le premier et le second auxiliaire de pliage (172, 174) sont extraits du pliage, que le premier et le second auxiliaire de pliage (172, 174) sont déplacés de sorte que le pliage se situe entre le premier et le second auxiliaire de pliage (172, 174) et est fixé par l'exercice d'une force, et que les éléments de préhension (188, 190, 192, 194) sont déclenchés et sont déplacés pour une nouvelle saisie de la première et de la seconde couche du tuyau pour réaliser un nouveau pliage.

5. Procédé selon au moins l'une des revendications précédentes,
**caractérisé en ce**
**que** la fixation est sélectionnée parmi le groupe pincement par déplacement relatif des auxiliaires de fixation, déplacement relatif des préhenseurs ou entraîneurs, pincement par élément de pincement séparé ou attraction de la couche par dépression sur au moins un des auxiliaires de fixation.

6. Dispositif de pliage (10 ; 66 ; 106) pour fabriquer un revêtement, tel qu'un film enveloppant, avec pliage télescopique, comprenant au moins un premier auxiliaire de pliage (12, 14 ; 70, 72 ; 108 ; 172, 174) dans lequel ou au moyen duquel une extrémité (42 ; 86 ; 102 ; 216) d'un tuyau (44 ; 68 ; 104 ; 218) composé d'un matériau flexible pour fabriquer le pliage est insérable ou enfilable partiellement à plusieurs reprises, sachant que le dispositif de pliage (10 ; 66 ; 106) présente un moyen de fixation (12, 14 ; 70, 72 ; 172, 174) au moyen duquel l'extrémité (42 ; 86 ; 102 ; 216) du tuyau (44 ; 68 ; 104, 218) ou au moins un pliage est fixable sur ledit au moins un auxiliaire de pliage (12, 14 ; 70, 72 ; 108 ; 172, 174), sachant que le moyen de fixation (14 ; 72 ; 108 ; 172, 174) est un second auxiliaire de pliage, sachant que le premier et le second auxiliaire de pliage (12, 14 ; 70, 72 ; 108 ; 172, 174) sont déplaçables relativement l'un par rapport à l'autre,
**caractérisé en ce**
**que** le dispositif de pliage (10 ; 66 ; 106) est conçu pour plier un revêtement (44) présentant des applications, sachant que le premier et/ou le second auxiliaire de pliage (12, 14 ; 70, 72 ; 108 ; 172, 174) est conçu en forme de fourche ou de U ou sachant que le premier et/ou le second auxiliaire de pliage (12, 14 ; 70, 72 ; 108 ; 172, 174) présentent des évidements adaptés aux applications pour offrir suffisamment de place pour les applications du revêtement (44) à l'état plié.

7. Dispositif de pliage selon la revendication 6,
**caractérisé en ce**
**que** l'extrémité (42 ; 86 ; 216) du tuyau (44 ; 68, 218) ou ledit au moins un pliage sont fixables entre les auxiliaires de pliage (12, 14 ; 70, 72 ; 172, 174) ou sur un des auxiliaires de pliage (12, 14 ; 70, 72 ; 172, 174), sachant que le tuyau (44 ; 68 ; 218) est enfilable sur les auxiliaires de pliage (12, 14 ; 70, 72 ; 172, 174) pour fabriquer le pliage, par un mouvement relatif entre les auxiliaires de pliage (12, 14 ; 70, 72 ; 172, 174) et le tuyau (44 ; 68 ; 216) contre une butée (50 ; 220, 222) d'au moins un des auxiliaires de pliage (12, 14 ; 70, 72 ; 172, 174) et/ou que de préférence, l'extrémité (102) du tuyau (104) est enfilable sur le premier et/ou le second auxiliaire de pliage (70, 72), sachant que les auxiliaires de pliage (70, 72) sont conçus écartables pour ouvrir l'extrémité (102), et sachant que le tuyau (104) est insérable dans un espace sous-tendu par les auxiliaires de pliage, par un mouvement relatif entre les auxiliaires de pliage (70, 72) et le tuyau (104) contre au moins une butée (98, 100) du premier ou du second auxiliaire de pliage (70, 72)

8. Dispositif de pliage selon la revendication 6 ou 7,
**caractérisé en ce**
**que** les auxiliaires de pliage (12, 14 ; 70, 72 ; 172, 174) sont déplaçables relativement l'un par rapport à l'autre, que l'extrémité (42 ; 86 ; 216) du tuyau (44 ; 68 ; 218) ou au moins un premier pliage entre les auxiliaires de pliage est fixable ou que l'extrémité (42 ; 86) du tuyau (44 ; 68) ou au moins un premier pliage est fixable par écartement des auxiliaires de pliage (70, 72) et/ou que de préférence, ledit au moins un auxiliaire de pliage (12, 14 ; 70, 72 ; 108 ; 172, 174) présente des moyens de dépression comme moyen de fixation, qui sont reliés à une source de dépression, sachant que les moyens de dépression sont conçus sous forme d'embouchures dans une surface dudit au moins auxiliaire de pliage (12, 14 ; 70, 72 ; 108) avec lesquelles le tuyau ou une couche (48, 52) du tuyau est fixable.

9. Dispositif de pliage selon au moins l'une des revendications 6 à 8,
**caractérisé en ce**
**que** le premier et/ou second auxiliaire de pliage (12, 14 ; 70, 72 ; 108 ; 172, 174) est couplé à un entraînement (40, 88) qui est entraîné de préférence mécaniquement, magnétiquement, électriquement, pneumatiquement et/ou hydrauliquement et maintenu dans une position quelconque.

10. Dispositif de pliage selon au moins l'une des revendications 6 à 9,
**caractérisé en ce**
**que** ledit au moins un premier et/ou second auxiliaire de pliage conçu en forme de U comprend deux branches (16, 18 ; 32, 34 ; 110, 112) avec respectivement un bord extérieur (20, 22), sachant que les bords extérieurs sont parallèles entre eux et définissent la largeur W, et sachant qu'une longueur des branches (16, 18 ; 32, 34 ; 110, 112) et/ou l'écart des branches (16, 18 ; 32, 34 ; 110, 112) entre elles est réglable de manière télescopique.

11. Dispositif de pliage selon au moins l'une des revendications 6 à 10,
**caractérisé en ce**
**que** les branches (16, 18 ; 32, 34 ; 110, 112) partent d'une traverse (24 ; 36) et que les branches (16, 18 ; 32, 34 ; 110, 112) sont disposées dans la traverse (24 ; 36) de manière à pouvoir coulisser le long d'un axe longitudinal de celle-ci et/ou que de préférence, le premier et/ou second auxiliaire de pliage (12, 14 ; 70, 72 ; 108) est disposé de manière déplaçable le long d'un élément d'appui (26 ; 74), sachant qu'au moins un des auxiliaires de pliage (12, 14 ; 70, 72 ; 108) est couplé à un entraînement pneumatique ou hydraulique (40).

12. Dispositif de pliage selon au moins l'une des revendications 6 à 11,
**caractérisé en ce**
**que** le dispositif de pliage (10) présente des premiers et seconds éléments de préhension (188, 190 ; 192, 194) qui sont conçus respectivement pour saisir une première et une seconde couche (224, 226) du tuyau (218) et que les éléments de préhension (188, 190 ; 192, 194) sont disposés de manière déplaçable dans le sens longitudinal et/ou transversal par rapport au premier et au second auxiliaire de pliage (172, 174) pour enfiler la première et la seconde couche (224, 226) du tuyau (218) sur les auxiliaires de pliage (172, 174).

13. Dispositif de pliage selon au moins l'une des revendications 6 à 12,
**caractérisé en ce**
**que** le premier et le second auxiliaire de pliage (172, 174) présentent respectivement des évidements correspondant entre eux (184, 186) qui sont conçus ouverts, de préférence en forme de fentes en direction d'une butée (220, 222) du premier et du second auxiliaire de pliage (172, 174) et/ou que notamment les évidements (184, 186) sont conçus pour correspondre aux préhenseurs (196, 198 ; 200, 202) des éléments de préhension (188, 190 ; 192, 194) et/ou que notamment les préhenseurs (196, 198 ; 200, 202) sont conçus de manière déplaçables l'un contre l'autre pour fixer les couches (224, 226) du tuyau (218) dans la zone des évidements (184, 186) et/ou que notamment les auxiliaires de pliage (172, 174) sont disposés de manière déplaçable par rapport aux éléments de préhension dans le sens longitudinal et transversal et/ou que notamment les préhenseurs sont conçus sous forme de pinces aspirantes.

14. Dispositif de pliage selon au moins l'une des revendications 6 à 13,
**caractérisé en ce**
**que** les éléments de préhension (188, 190, 192, 194) sont disposés de manière déplaçable le long de rails (210, 212) par rapport aux auxiliaires de pliage (172, 174), notamment au-dessus ou en dessous des auxiliaires de pliage (172, 174) et/ou que les éléments aspirants (196, 198 ; 200, 202) sont disposés de manière déplaçable les uns contre les autres.

15. Dispositif de pliage selon au moins l'une des revendications 6 à 14,
**caractérisé en ce**
**que** le dispositif de pliage présente un moyen (46 ; 88) de créer un courant d'air, sachant qu'un orifice de courant d'air est conçu pour introduire le courant d'air dans l'extrémité (42 ; 86 ; 102) du tuyau en film (44 ; 68 ; 104), fixée ou sous-tendue entre les éléments de pliage (70, 72) et/ou que notamment le tuyau est composé d'un matériau flexible sélectionné parmi le groupe comprenant film plastique, papier, Tyvec (marque déposée), non-tissé, matière tissée.

16. Dispositif de pliage selon au moins l'une des revendications 6 à 15,
**caractérisé en ce**
**que** le premier et le second auxiliaire de pliage (12, 14 ; 70, 72 ; 108) présentent de préférence un repère au moyen duquel sont définies les différentes profondeurs de pliage et/ou que notamment le repère est apposé sous forme de graduation sur une surface du premier et du second auxiliaire de pliage et/ou que le repère est conçu sous forme de repère haptique tel qu'une encoche latérale ou une rainure en superficie et/ou que le repère est conçu sous forme de repère optique tel qu'une diode électroluminescente, une bande lumineuse et/ou un marquage laser.

17. Dispositif de pliage selon au moins l'une des revendications 6 à 16,
**caractérisé en ce**
**que** le premier et/ou le second auxiliaire de pliage (12, 14 ; 70, 72, 108) présentent sur une surface tournée vers le second ou le premier auxiliaire de pliage (12, 14 ; 70, 72 ; 108) au moins un élément d'espacement au moyen duquel l'extrémité du tuyau, ladite au moins une couche du tuyau et/ou ledit au moins un pliage du tuyau est fixable contre le second ou le premier auxiliaire de pliage et/ou que ledit en particulier au moins un élément d'espacement est composé d'un matériau sélectionné parmi le groupe comprenant silicone, plastique souple, plastique dur et/ou métal, et/ou que ledit en particulier au moins un élément d'espacement est conçu de préférence sous forme de point, de bande ou sous forme ondulée et/ou que l'élément d'espacement est conçu sous forme d'aiguille.

18. Dispositif de pliage selon au moins l'une des revendications 6 à 17,
**caractérisé en ce**
**que** le premier, deuxième et/ou troisième auxiliaire de pliage sont guidés par un équipement de manipulation tel qu'un robot.
